(19) Europäisches Patentamt
European Patent Office
Office européen des brevets

(11) Publication number: **0 001 238**
**B1**

(12) # EUROPEAN PATENT SPECIFICATION

(45) Date of publication of patent specification: **04.07.84**

(21) Application number: **78100823.0**

(22) Date of filing: **05.09.78**

(51) Int. Cl.³: **C 07 D 233/78,**
C 07 D 233/76,
C 07 D 233/90,
C 07 D 403/12,
C 07 D 403/06,
A 61 K 31/415

(54) **Hydantoin derivatives, their synthesis, pharmaceutical formulations and intermediates in their preparation.**

(30) Priority: **05.09.77 GB 3705477**
**01.12.77 GB 5009277**
**31.05.78 GB 3705478**

(43) Date of publication of application:
**04.04.79 Bulletin 79/7**

(45) Publication of the grant of the patent:
**04.07.84 Bulletin 84/27**

(84) Designated Contracting States:
**BE CH DE FR NL SE**

(56) References cited:
**FR - A - 2 362 839**
**GB - A - 1 346 279**

(73) Proprietor: **THE WELLCOME FOUNDATION LIMITED**
**183-193 Euston Road**
**London NW1 2BP (GB)**

(72) Inventor: **Whittaker, Norman**
**37 Brabourne Rise**
**Beckenham Kent (GB)**
Inventor: **Caldwell, Albert Gordon**
**119 Gates Green Road**
**West Wickham Kent (GB)**

(74) Representative: **Berg, Wilhelm, Dr. et al,**
**Dr. Berg, Dipl.-Ing. Stapf, Dipl.-Ing. Schwabe, Dr. Dr. Sandmair Patentanwälte Postfach 860245**
**D-8000 München 86 (DE)**

**EP 0 001 238 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Hydantoin derivatives, defined hereinbelow in formula (I), have been found to have pharmacological properties related to those of natural prostaglandins, as demonstrated by their ability to mimic or antagonise the physiological effects of the natural prostaglandins in various biological preparations. In particular, certain compounds of formula (I) have been found to be potent mimetics of the anti-platelet aggregatory properties of prostaglandin $E_1$.

The invention therefore relates to compounds of formula (I):

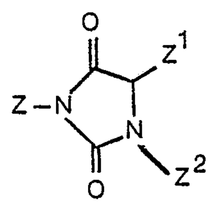

(I)

wherein Z is hydrogen or alkyl; one of $Z^1$ and $Z^2$ is represented by the group —X—$X^1$—$X^2$—$X^3$

wherein X is methylene, $X^1$ is phenylene, —C≡C—, *cis* or *trans* —CH=CH— or —$CH_2$—$CQ_2$— in which each Q is independently selected from hydrogen and alkyl or the two Q's together form an alkylene radical having four, five or six carbon atoms; $X^2$ is a covalent bond or a straight or branched alkylene chain having 1 to 6 carbon atoms; $X^3$ is selected from 5-tetrazolyl, carboxyl, carbamoyl, hydroxymethylene and alkoxycarbonyl; and one of the methylene groups of X, $X^1$ and $X^2$ may be replaced by oxa (—O—) or thia (—S—) provided that at least one carbon atom separates the oxa or thia group from a —C≡C—, —CH=CH— or —CO— group or from the nitrogen of the hydantoin ring;

and the other of $Z^1$ and $Z^2$ is represented by the group —Y—$Y^1$—$Y^2$—$Y^3$

wherein Y is —$CR_2$—$CH_2$— in which each R is independently selected from hydrogen and methyl; $Y^1$ is carbonyl, methylene substituted by hydroxyl or methylene substituted by hydroxyl and alkyl; $Y^2$ is a covalent bond or straight or branched alkylene having 1 to 7 carbon atoms optionally substituted on the carbon adjacent $Y^1$ by one or two groups each of which may be alkyl or a cyclic radical; $Y^3$ is hydrogen, hydroxy, alkoxy having 1 to 7 carbon atoms, a cyclic radical, phenyl, benzyl, phenoxy or benzyloxy, wherein each of phenyl, benzyl, phenoxy and benzyloxy may be substituted in the benzene ring by one or more groups selected from hydroxy, halo, nitro, amino, acylamino, alkenyl, alkoxy, phenyl, benzyloxy and alkyl which may itself be substituted by one or more halo groups; or $Y^2$ and $Y^3$ together from an alkyl group having 1 to 7 carbon atoms of which at least one hydrogen is replaced by fluoro; or Y is a bond, —$CH_2$—, or —$CH_2 \cdot CH_2$— and $Y^1$, $Y^2$ and $Y^3$ taken together form a cycloalkyl or bicycloalkyl group substituted by a hydroxyl group; provided that:

(A) either

(i) X or $X^1$ includes an oxa group; or

(ii) one of X, $X^1$ and $X^2$ includes a thia group; or

(B) when the values of X, $X^1$ and $X^2$ are other than as defined in A, then

(i) at least one of $Y^2$ and $Y^3$ includes a cyclic radical other than an unsubstituted cycloalkyl group having from 3 to 10 carbon atoms or an unsubstituted bicycloalkyl group having from 4 to 10 carbon atoms; or

(ii) $Y^2$ and $Y^3$ taken together form an alkyl group of from 1 to 7 carbon atoms of which at least one hydrogen is replaced by fluoro; or

(iii) $Y^1$, $Y^2$ and $Y^3$ taken together form a hydroxy-substituted bicycloalkyl group; or

(iv) $Y^3$ includes a benzene ring substituted by benzyloxy:

in which the term cyclic radical means the monovalent radical derived by loss of a ring hydrogen atom from a monocyclic or polycyclic compound having from 3 to 12 ring atoms selected from carbon, nitrogen, oxygen and sulphur, which compound may be saturated or unsaturated and may be further substituted by one or more alkyl groups, but excluding phenyl.

According to a preferred embodiment the invention relates to a compound of the formula (I) wherein Z is hydrogen

One of $Z^1$ and $Z^2$ is a group

—X—$X^1$—$X^2$—$X^3$ wherein

X is methylene

$X^1$ is —C=C—, *cis* or *trans* —CH=CH— or —$CH_2$—$CH_2$—;

$X^2$ is a covalent bond or a straight or branched alkylene chain having 1 to 6 carbon atoms optionally having one of its methylene groups replaced by oxa(—O—) or thia (—S—) provided that at least one carbon atom separates the oxa or thia from a —C=C—, —CH=CH— or —O— group; and

$X^3$ is selected from carboxyl, carbamoyl, hydroxymethylene and alkoxycarbonyl;

and the other of $Z^1$ and $Z^2$ is a group

—Y—$Y^1$—$Y^2$—$Y^3$ wherein

Y, $Y^1$, $Y^2$ and $Y^3$ are as defined above. More preferred are the compounds of formula (I) wherein

Z is hydrogen

$Z^1$ is a group

$—X—X^1—X^2—X^3$ wherein

X is methylene

$X^1$ is *cis* or *trans* —CH=CH— or $—CH_2—CH_2—$

$X^2$ is $—CH_2.CH_2—$, $—CH_2.O.CH_2—$ or $—CH_2.S.CH_2—$; and

$X^3$ is carboxyl or alkoxycarbonyl; and

$Z^2$ is a group

$—Y—Y^1—Y^2—Y^3$ wherein

Y is $—CH_2—CH_2—$

$Y^1$ is methylene substituted by hydroxyl or methylene substituted by hydroxyl and alkyl;

$Y^2$ is a covalent bond or straight or branched alkylene having 1 to 7 carbon atoms optionally substituted on the carbon adjacent $Y^1$ by one or two groups each of which may be alkyl or a cyclic radical;

$Y^3$ is hydrogen, alkoxy of 1 to 7 carbon atoms or a cyclic radical; the most preferred compounds of which are those wherein the cyclic radical is cycloalkyl of 3 to 10 carbon atoms, such as cyclopentyl or cyclohexyl.

The invention as well covers the diastereomers of said compounds which diastereomers are shown to be the less polar by thin layer chromatography using silicia gel and a solvent system of chloroform:methanol:acetic acid in the proportions of 90:5:5.

The invention as well encloses a method for the preparation of the compounds defined above, pharmaceutical compositions comprising these compounds for use in the inhibition of aggregation of blood platelets, in the treatment or prophylaxis of thrombosis in a mammal or mammalian tissue and of gastric lesions in a mammal and for use in the lowering of blood pressure in a mammal including man, and intermediates for the preparation of the compounds defined above. Preferably said composition comprises from 0.1 to 50 mg of a compound of formula (I).

In formula (I), the term cyclic radical means the monovalent radical derived by loss of a ring hydrogen atom from a monocyclic or polycyclic compound having from 3 to 12 ring atoms selected from carbon, nitrogen, oxygen and sulphur, which compound may be saturated or unsaturated and may be further substituted by one or more alkyl groups, but excluding phenyl. Such cyclic radicals include cycloalkyl having 3 to 10 carbon atoms such as cyclopropyl, cyclopentyl, cyclohexyl and cyclooctyl, bicycloalkyl having 4 to 10 carbon atoms such as adamantyl or norbornyl (bicyclo[2,2,1]heptyl), spiroalkanyl having 5 to 12 carbon atoms such as 2-spiro[3,3]heptyl, 1-spiro[4,4]nonyl and 8-spiro[4,5]decyl, cycloalkenyl having 4 to 10 carbon atoms such as 4-cyclopentenyl, heterocyclic radicals such as tetrahydrofuranyl and tetrahydropyranyl and heteroaryl radicals such as thienyl, furyl, pyridyl, pyrimidyl, thiazolyl, imidazolyl and diazapinyl. Included in the term cyclic radical are those wherein one or more hydrogen atoms are replaced by fluoro.

Unless otherwise stated, in formula (I) and other formulae in this specification, alkyl moieties are selected from methyl, ethyl, propyl, butyl, pentyl and hexyl, including all isomers thereof; for example, in the definitions of $Y^1$ and $Y^2$ the alkyl groups are preferably methyl; and the alkyl moiety of alkoxycarbonyl is desirably methyl or ethyl. Similarly alkenyl groups preferably have 2 to 4 carbon atoms for example vinyl.

In a compound of formula (I) the bonding of the divalent phenylene group may be *ortho, meta* or *para,* and the oxa or thia group is preferably adjacent the phenylene or when $X^1$ is other than phenylene then $X^2$ may be $—CH_2—O—CH_2—$ or $—CH_2—S—CH_2—$.

Included in the meaning of compounds of formula (I) are the salts corresponding to the carboxylic acids and tetrazoles when $X^3$ is carboxyl or tetrazolyl respectively, and the salts which may also be formed when Z is hydrogen. Particularly valuable salts for medical purposes are those having a pharmaceutically acceptable cation such as ammonium or that of an alkali metal e.g. sodium and potassium, an alkaline earth metal e.g. calcium and magnesium, or an organic base, particularly an amine such as ethanolamine. Salts having non-pharmaceutically acceptable cations are included within the ambit of this invention as useful intermediates to pharmaceutically acceptable salts, or the acids or esters of formula (I).

Except when there is clear indication to the contrary, formula (I) and other formulae in the specification embrace all stereoisomers represented therein. In particular such formulae include the enantiomeric forms, such mixtures as are designated racemates, and diastereoisomers.

The compounds of formula (I) may be synthesised by any method known in the art for the synthesis of compounds of analogous structure. For example, they may be prepared from the corresponding derivatives of hydantoic acid of formula (II):

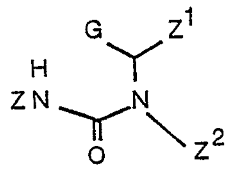

(II)

wherein G is carboxyl or a derivative thereof such as amide or ester in particular an alkyl ester, and each of Z, $Z^1$ and $Z^2$ has the same meaning as in formula (I), by cyclisation under acidic conditions or by heating alone. The reaction may be effected in the absence of a solvent, but if desired an inert solvent may be used, for example a hydrocarbon such as petrol. Alternatively, where G is alkoxycarbonyl, cyclisation may be effected in the presence of a suitable base, for example an alkoxide such as sodium ethoxide.

Compounds of formula (II) are conveniently prepared from an amino acid derivative of formula (III):

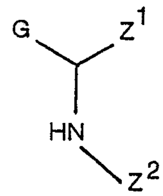

(III)

wherein G, $Z^1$ and $Z^2$ are as defined in formula (I) provided that G may also be nitrile, by reaction with cyanic acid or an alkyl *iso*-cyanate depending respectively on whether Z is hydrogen or alkyl.

When cyanic acid is used, the cyanic acid is conveniently produced *in situ* by the use of an alkali metal cyanate, e.g. potassium cyanate, and an acid which may be present as an acid addition salt of the compound of formula (III) or a free acid of formula (III) wherein either or both of R and $X^3$ is hydrogen. Alternatively an equivalent amount of mineral acid or an organic acid may be added to the reaction medium. The reaction may proceed in the absence of a solvent but desirably an inert solvent is used which is preferably polar such as water or a mixture of water with acetone, dimethylformamide, dimethylsulphoxide or a lower alkanol such as ethanol or it may be a hydrocarbon, an ether or halogenated hydrocarbon such as chloroform. Where desired, for example if no solvent is used, the reaction may be promoted by heating the reactants.

Similar reaction conditions may be used when an alkyl *iso*-cyanate is used except that it is unnecessary to provide an equivalent amount of acid, as an acid addition salt or otherwise, in the reactants.

Instead of using a cyanate or isocyanate, a compound of formula (III) may be reacted with urea, nitrourea or an *N*-alkylurea as appropriate. A solvent is not essential but if desired an inert solvent such as one mentioned above may be used, and the reaction is preferably effected at an elevated temperature, for example from 100°C to 125°C but temperatures up to 150°C may be employed.

In the above described synthesis, the intermediates of formula (II) need not be isolated from the reaction mixture and may be converted directly to compounds of formula (I) under the described reaction conditions.

An intermediate of formula (III) may be conveniently prepared by reaction of a compound of formula (IV) with a compound of formula (V):

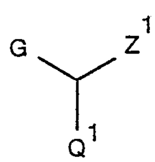

(IV) $Q^2-Z^2$ (V)

wherein G, $Z^1$ and $Z^2$ are as defined in formula (III), one of $Q^1$ and $Q^2$ is amino and the other is halo, preferably bromo. The reaction may be carried out by heating in the absence of solvent or in the presence of an inert solvent such as ethanol.

The intermediates of formula (III) wherein $Z^2$ is —Y—$Y^1$—$Y^2$—$Y^3$ when $Y^1$ is carbonyl may also be prepared by reaction of an amine of formula (IV) wherein $Q^1$ is amino with an unsaturated ketone of formula (VI):

$$CR_2{=}CH.CO.Y^2.Y^3 \qquad (VI)$$

wherein $Y^2$ and $Y^3$ have the same meaning as in formula (III); the reaction being effected in the

presence or absence of an inert solvent, and at room temperature or optionally with heating.

Hydantoins of formula (I) may also be prepared by cyclisation of a compound of formula (VII):

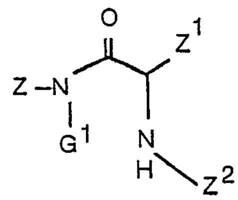

(VII)

wherein Z, $Z^1$ and $Z^2$ are as defined in formula (I) and $G^1$ is carboxyl or a reactive derivative thereof such as alkoxycarbonyl e.g. ethoxycarbonyl. Compounds of formula (VII) may be cyclised under similar conditions as a compound of formula (II) and conveniently the method used to prepare a compound of formula (VII) is chosen such that the prevailing reaction conditions permit spontaneous cyclisation.

For example, the intermediates of formula (VII) may be prepared by reacting a compound of formula (V) with a compound of formula (VIII):

$Q^2 - Z^2$ (V)

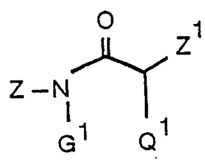

(VIII)

wherein one of $Q^1$ and $Q^2$ is halogeno, preferably chloro or bromo and the other is amino and each of Z, $Z^1$, $Z^2$ and $G^1$ have the same meaning as in formula (VII). The reaction may be effected by admixture of the reactants or optionally an inert solvent is used and the mixture is heated. Suitable solvents include alkanols, ethers, hydrocarbons and halogenated hydrocarbons.

The compounds of formula (VIII) may themselves be made by reacting an appropriate carbamic acid derivative, for example an alkyl ester, with a compound of formula (IV), using techniques known to those skilled in the art.

In a method related to those described hereinbefore, the hydantoins of formula (I) may be prepared by reacting a compound of formula (IX):

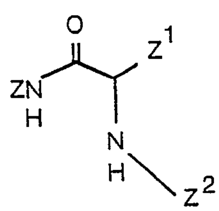

(IX)

wherein each of Z, $Z^1$ and $Z^2$ has the same meaning as in formula (I) with a carbonic acid derivative. Any carbonic acid derivative known to those skilled in the art as appropriate may be used, for example, phosgene, diphenylcarbonate or an alkyl haloformate such as ethyl chloroformate. The reaction is desirably effected in the presence of a base, for example an amine such as triethylamine or di-*iso*-propyl ethylamine, and using an inert aprotic solvent such as toluene, dimethylformamide or an ether such as diethylether. The reaction may be carried out at room temperature but if desired the reaction mixture may be heated.

The intermediates of formula (IX) may be made using methods analogous to those described above for the preparation of compounds of formula (III).

The hydantoins of formula (I) wherein Z is alkyl may also be prepared by alkylation, using an alkylating agent which may be designated as a reactive ester derivative of an alcohol $J^3$.OH, of a compound of formula (X):

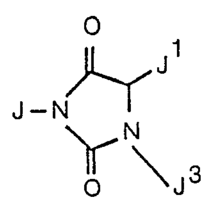

(X)

wherein J is hydrogen or alkyl, $J^1$ is hydrogen or $Z^1$, $J^2$ is hydrogen or $Z^2$ and $J^3$ is alkyl, $Z^1$ or $Z^2$,

provided that one of J, $J^1$ and $J^2$ is hydrogen and $J^3$ does not have the same value as J, $J^1$ or $J^2$; in the definition of $J^1$, $J^2$ and $J^3$ each of $Z^1$ and $Z^2$ has the same meaning as in formula (I). Suitable reactive ester derivatives include chloride, bromide, iodide and sulphonates such as *p*-toluenesulphonate, methanesulphonate and benzenesulphonate. The alkylation may be effected using reaction conditions which are known in the art to be suitable, for example in the presence of a base such as an alkalimetal hydride, alkali metal amide, or alkalimetal alkoxide, typically sodium hydride or a sodium alkoxide e.g. sodium methoxide.

The reaction is conveniently carried out in an inert solvent which simply acts as a diluent for the reactants such as toluene, dioxan, ether, dimethylformamide, tetrahydrofuran, dimethylsulphoxide or acetonitrile or when the base is an alkali metal alkoxide then the corresponding alkanol may be used.

It will be appreciated that the intermediates of formula (X) wherein J is hydrogen are also compounds of formula (I) and may be prepared by one of the foregoing meethods. The compounds of formula (X) may further be prepared by adaptation of methods already known in the art (see for example Chemical Reviews (1950) *46*, p. 403—425).

A further preparation of compounds of formula (I) is by reduction of a corresponding unsaturated compound of formula (XI):

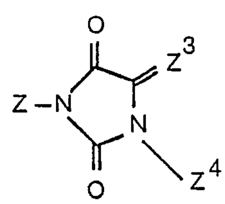

(XI)

wherein either $Z^3$ is $=CR—CH_2—Y^1—Y^2—Y^3$ and $Z^4$ is $—X—X^1—X^2—X^3$ or $Z^3$ is $=CH—X^1—X^2—X^3$ and $Z^4$ is $—Y—Y^1—Y^2—Y^3$ in which each of R, X to $X^3$ and Y to $Y^3$ is as defined in formula (I), with a suitable reducing agent.

A suitable reducing agent is stannous chloride which may be used as an aqueous solution optionally in the presence of dilute mineral acid or catalytic hydrogenation may be effected in the presence of for example Raney nickel, platinum, palladium, ruthenium or rhodium. The choice of reducing agent in a given situation will of course be dictated by the presence of other reactive groups in the molecule which may themselves be susceptible to reduction.

The intermediates of formula (XI) may be prepared by the following series of reactions:

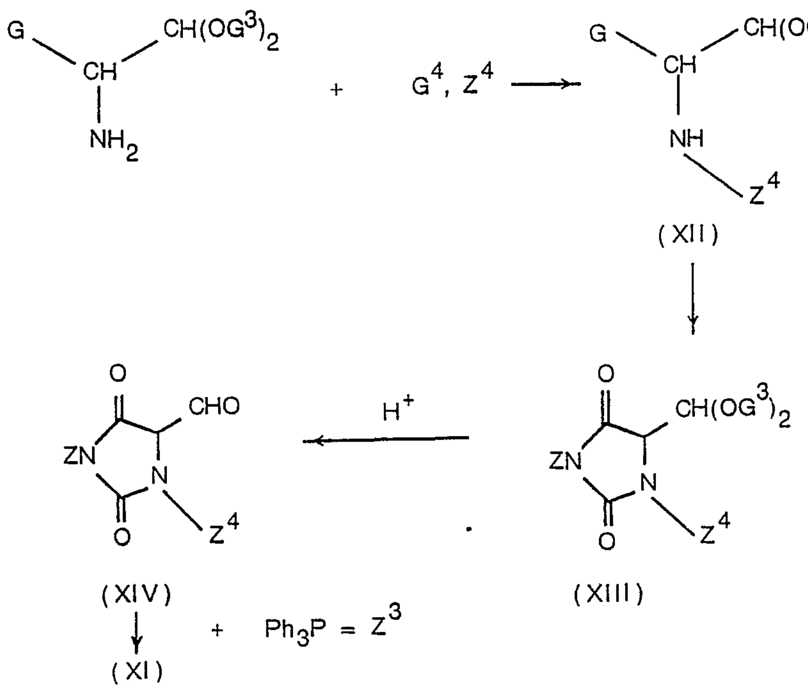


(XII) (XIII) (XIV) (XI)

In the above formulae Z, $Z^3$, $Z^4$ and G have the same meaning as in formula (XI) and (III) respectively, $G^3$ is alkyl for example *n*-butyl and $G^4$ is halo such as bromo. The formation of (XIII) is analogous to the ring closure involving a compound of formula (II) and compounds of formula (XIV), are prepared using concentrated mineral acid such as hydrochloric acid.

Tetrazoles of formula (I) may be prepared from corresponding compounds wherein the group —$X^3$ is replaced by

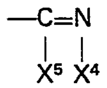

wherein $X^4$ and $X^5$ together form a bond (nitrile), $X^4$ is hydrogen or alkyl and $X^5$ is alkoxy (imidoester), alkylthio (imidothioester), —NH—$NH_2$ (amidrazone), or amino (amidine) or $X^4$ is hydroxy and $X^5$ is amino (amidoxime). The reaction is preferably carried out in a polar aprotic liquid medium such as dimethylformamide using a salt of hydrazoic acid e.g. sodium azide. However, when $X^3$ is replaced by an amidine or amidrazone, a suitable reagent is nitrous acid. If an amidine is reacted with nitrous acid then reduction of the intermediate nitrosation product, with or without prior isolation, using for example sodium amalgam is required to give the corresponding tetrazole. The tetrazole precursor may be obtained by well known methods, for example the nitrile may be obtained by dehydration of the corresponding amide.

The alcohols of formula (I) wherein $X^3$ is hydroxymethylene may also be obtained by reduction with an appropriate reducing agent of the corresponding acid, ester, acid halide, acid anhydride or aldehyde. The appropriate reducing agent will depend on the particular substrate, but reactants which may be used are sodium in ethanol. In particular a carboxylic acid may for example be converted to a corresponding mixed anhydride with ethylchloroformate in the presence of a base such as triethylamine, and subsequently reduced to the alcohol using sodium borohydride. Similarly an ester may be reduced to the alcohol using di-*iso*-butyl aluminium hydride in an inert solvent such as ether or hydrocarbon such as hexane or benzene. Such alcohols may also be prepared by catalytic hydrogenation.

Alternatively the alcohols of formula (I) wherein $X^3$ is hydroxymethylene may be prepared by hydrolysis of a corresponding halide with an appropriate reagent. For this purpose a hydroxide may be used for example an aqueous alkali or a suspension of silver oxide in water.

In the synthesis of hydantoins of formula (I) having a hydroxyl group in a side chain it may be desirable to protect this during the course of the reaction. This may be readily effected in known manner using a protecting group such as acyl, aroyl, tetrahydropyran-2-yl, 1-ethoxyethyl or aralkyl, for example benzyl.

Removal of protecting groups may be carried out by appropriate methods known to those skilled in the art: for example an acyl group may be removed by acid or base hydrolysis, and a benzyl group by reductive cleavage.

Furthermore a ketone of formula (I) wherein $Y^1$ is carbonyl may be converted to the corresponding secondary alcohol by reduction with a suitable reducing agent, such as sodium borohydride. Also, an alcohol of formula (I) wherein $Y^1$ is —CH.OH— may be oxidised to the corresponding ketone using Jones' reagent, acid dichromate or any other suitable reagent.

Similarly where the compounds of formula (I) have a C≡C or CH=CH bond these may be converted by conventional hydrogenation techniques, for example using a Lindlar type or Adams catalyst, to the corresponding ethylenic or saturated compounds as appropriate.

The hydantoins of formula (I) have an asymmetric 5-carbon atom, and a further asymmetric centre is present in those compounds wherein $Y^1$ includes a hydroxyl group. Such alcohols therefore exist as four isomers which are separable by thin layer chromatography or high performance liquid chromatography into two diastereomers, each of which is a racemic mixture of two isomers. On separation of the diastereomers, one diastereomer may be converted to a mixture of the four isomers by treatment with a base, such as an alkali metal hydroxide, and subsequently re-separated to provide two diastereomers. Repeated use of this technique enables the conversion of one diastereomer to the other; this may be desirable when one diastereomer has a preferred biological activity.

The corresponding alcohols of formula (III) also exist in four isomeric forms. If desired, these may be separated into two epimers and subsequent cyclisation to a compound of formula (I) retains the stereochemical configuration.

In all of the foregoing chemical procedures it is of course evident that the choice of reactant will be dictated in part by the functional groups present in the substrate, and where necessary reactants having an appropriate selectivity of action must be used.

The hydantoins of formula (I) are of value in having pharmacological properties related to those of natural prostaglandins; that is, the hydantoins mimic or antagonise the biological effects of members of the prostaglandin (PG) 'A', 'B', 'C', 'D', 'E' and 'F' series. For example, hydantoins of formula (I) have been found to mimic the antiaggregatory effect of $PGE_1$ on blood platelets, and to antagonise the contraction induced by $PGE_2$ or $PGF_2$ on smooth muscle taken from the rat stomach, rat colon, chick rectum and guinea pig trachea. In general, antagonistic properties, as opposed to mimetic, have been observed when using larger doses of the hydantoins. The pharmacological profile, by which is meant the relative activities, mimetic or antagonistic, compared with the natural prostaglandins, will of course vary depending on the specific hydantoin under consideration.

By reason of their prostaglandin-related properties, the hydantoins of formula (I) are useful in the pharmacological characterisation and differentiation of the biological activities of the natural prostaglandins and their "receptors". The further understanding of the physiological role of prostaglandins is of course valuable in the search for new and improved therapeutic substances.

The hydantoins of formula (I) are also of value as therapeutic agents. In particular hydantoins such as those described previously as having a potent anti-aggregatory effect on blood platelets are useful whenever it is desired to inhibit platelet aggregation or to reduce the adhesive character of platelets, and may be used to treat or prevent the formation of thrombi in mammals, including man. For example, the compounds are useful in the treatment and prevention of myocardial infarcts, to treat and prevent thrombosis, to promote patency of vascular grafts following surgery, and to treat complications of arteriosclerosis and conditions such as atherosclerosis, blood clotting defects due to lipermia, and other clinical conditions in which the underlying aetiology is associated with lipid imbalance or hyperlipidemia. A further use for such compounds is as an additive to blood and other fluids which are used in artificial extra-corporeal circulation and perfusion of isolated body portions.

A group of compounds which are particularly valuable as inhibitors of platelet aggregation are those of formula (I) wherein Z is hydrogen; $Z^1$ is carboxyalkyl wherein the alkylene moiety has 3 to 9 carbon atoms; and $Z^2$ is a group $—(CH_2)_2.CH.OH.Y^2.Y^3$ wherein $Y^2$ is a bond or branched alkylene having a tertiary carbon atom adjacent the hydroxy-substituted carbon and $Y^3$ is as defined in formula (I).

Within this group of compounds, those wherein $Z^1$ is carboxyhexyl and $Y^3$ is cycloalkyl having 4 to 7 carbon atoms are especially active.

The hydantoins of formula (I) also cause relaxation of vascular smooth muscle in a similar way as do members of the prostaglandin 'A' and 'E' series. Compounds relaxing vascular smooth muscle are capable of inducing vasodilation and therefore have anti-hypertensive properties and are useful in lowering the blood pressure in mammals, including man, and may be used alone or in combination with a $\beta$-adrenoceptor blocking agent or another antihypertensive substance for the treatment of all grades of hypertension including essential, malignant and secondary hypertension.

The compounds of formula (I) also mimic the effect of $PGE_1$ of antagonising histamine induced broncho-constriction. Compounds (I) having this property may be used in the treatment or prophylaxis of bronchial asthma and bronchitis by alleviating the bronchoconstriction associated with this condition.

Hydantoins of formula (I), which inhibit pentagastrin-induced gastric acid secretion and reduce the formation of aspirin-induced gastric lesions in rats are useful in reducing excessive gastric secretion. reducing and avoiding gastro-intestinal ulcer formation and accelerating the healing of such ulcers already present in the gastrointestinal tract whether such ulcers arise spontaneously or as a component of polyglandular adenoma syndromes.

Intravenous infusions of hydantoins of formula (I), to dogs increase the urine volume indicating a potential utility for such compounds as diuretic agents, the uses of which include the treatment of oedema for example oedema associated with heart failure, liver failure or kidney failure in man or other mammals.

A further use for hydantoins of formula (I) which mimic the uterine smooth muscle effects of $PGE_2$ and $PGF_{2\alpha}$ is as antifertility agents, in particular as abortifacients.

In addition the compounds of formula (I) may be used in the treatment of proliferative skin diseases such as are associated with excessive cell division in the epidermis or dermis which may be accompanied by incomplete cell differentiation. Particular conditions which may be alleviated include psoriasis, atopic dermatitis, nonspecific dermatitis, primary irritant contact dermatitis, allergic contact dermatitis, basal and squamous cell carcinomas of the skin, lamellar icthyosis, epidermolytic hyperkeratosis, premalignant sun induced keratosis, non malignant keratosis, acne, and seborrheic dermatitis in humans and atopic dermatitis and mange in domestic animals. For the treatment of these conditions, the compounds are desirably applied topically to the affected skin. Alternatively they may be administered by an intradermal or intramuscular injection which may be directly into the skin lesion or into the surrounding tissue. Injectable compositions will generally contain from 0.1 to 0.5% w/v of active ingredient.

The amount of a compound of formula (I) required to achieve the desired biological effect will of course depend on a number of factors, for example, the specific compound chosen, the use for which it is intended, the mode of administration, and the recipient. In general, a daily dose may be expected to lie in the range of from 1 $\mu$g to 20 mg per kilogram bodyweight. For example, an intravenous dose may lie in the range of from 5 $\mu$g to 1 mg/kg which may conveniently be administered as an infusion of from 0.01 to 50 $\mu$g per kilogram per minute. Infusion fluids suitable for this purpose may contain from 0.001 to 100, for example from 0.01 to 10 $\mu$g per millilitre. Unit doses may contain from 10 $\mu$g to 100 mg of a compound of formula (I), for example ampoules for injection may contain from 0.01 to 1 mg, and orally administrable unit dose formulations such as tablets or capsules may contain from 0.1 to 50, for example 2 to 20 mg.

More specifically, when a compound of formula (I) is used to inhibit platelet aggregation it is generally desirable to achieve a concentration in the appropriate liquid, whether it be the blood of a patient or a perfusion fluid, of about 1 $\mu$g to 10 mg, for example from 10 $\mu$g to 1 mg, per liter.

The abovementioned doses refer to the acids, amides, esters, alcohols and tetrazoles of formula (I); where a salt is used, the dose should be taken as referring to the corresponding anion.

For use in the treatment or prophylaxis of the conditions referred to above, while the hydantoin compounds may be used as the raw chemical they are preferably presented with an acceptable carrier therefor as a pharmaceutical formulation. The carrier must of course be 'acceptable' in the sense of being compatible with the other ingredients of the formulation and not deleterious to the recipient thereof. The carrier may be a solid or a liquid, and is preferably formulated with a hydantoin compound as a unit-dose formulation, for example a tablet, which may contain from 0.05% to 95% by weight of the hydantoin compound. Other pharmacologically active substances may also be present in formulations of the present invention as indicated above. The hydantoin compounds may be incorporated in the formulations either in the form of the acid or the salt or ester thereof, and the formulations may be prepared by any of the well-known techniques of pharmacy consisting essentially of admixture of the components of the formulation.

The formulations include those suitable for oral, rectal, topical (buccal — e.g. sub-lingual), parenteral (that is subcutaneous, intramuscular and intravenous) administration, although the most suitable route in any given case will depend on the nature and severity of the condition being treated, and on the nature of the hydantoin compound.

Formulations suitable for oral administration may be presented as discrete units such as capsules, cachets, lozenges or tablets each containing a predetermined amount of hydantoin compound; as a powder or granules; as a solution or a suspension in an aqueous liquid or a non-aqueous liquid; as an oil-in-water emulsion; or as a water-in-oil liquid emulsion. Such formulations may be prepared by any of the methods of pharmacy but all methods include the step of bringing into association the hydantoin compound with the carrier which constitutes one or more accessory ingredients. In general they are prepared by uniformly and intimately admixing the hydantoin compound with liquid or finely divided solid carriers or both, and then, if necessary, shaping the product into the desired presentation. For example a tablet may be prepared by compression or moulding a powder or granules of the hydantoin compound, optionally with one or more accessory ingredients. Compressed tablets may be prepared by compressing, in a suitable machine, the hydantoin compound in a free-flowing form such as a powder or granules optionally mixed with a binder, lubricant, inert diluent, surface active or dispersing agent(s). Moulded tablets may be made by moulding in a suitable machine the powdered hydantoin compound moistened with an inert liquid diluent.

Formulations suitable for buccal (sub-lingual) administration include lozenges comprising a hydantoin compound in a flavoured basis, usually sucrose and acacia or tragacanth; and pastilles comprising a hydantoin compound in an inert basis such as gelatin and glycerin; or sucrose and acacia.

Formulations suitable for topical applications to the skin preferably take the form of an ointment, cream, lotion, paste, gel, spray, aerosol or oil. Carriers which may be used include Vaseline (registered Trademark), lanoline, polyethylene glycols, alcohols and combinations thereof. The active ingredient is generally present in a concentration of from 0.1 to 15% w/w of the composition, for example from about 0.5 to about 2%.

Formulations of the present invention suitable for parenteral administration conveniently comprise sterile aqueous preparations of a hydantoin compound, which preparations are preferably isotonic with the blood of the intended recipient. These preparations are preferably administered intravenously, although administration may also be effected by means of subcutaneous or intramuscular injection. Such preparations may be conveniently prepared by admixing the hydantoin compound with water and rendering the product sterile and isotonic with the blood.

Formulations suitable for rectal administration are preferably presented as unit-dose suppositories. These may be prepared by admixture of the hydantoin compound with one or more of the conventional solid carriers, for example cocoa butter, and shaping of the resulting mixture.

Reference Preparation 1

Preparation of 5-(6-Carboxylhexyl)-1-(3-hydroxy-4,4-dimethyl-5-phenylpentyl)hydantoin

*A. Diethyl 2-aminononanedioate*

Diethyl acetamidomalonate (16.7 g) and ethyl 7-bromoheptanoate (16.6 g) were dissolved in ethanolic ethoxide (prepared from sodium (1.51 g) and absolute ethanol (30 ml) and the mixture was refluxed for 27 hours. The cooled solution was poured into ice-water, the product was extracted into ether, and the dried extract was evaporated to give crude diethyl acetamido-(6-ethoxycarbonylhexyl)-malonate as a pale yellow oil, $\delta$ 2.2 (3H, singlet, $—COCH_3$), 4.17 (6H, multiplet, 3 $\times$ $—OCH_2—CH_3$). This amide was refluxed with concentrated hydrochloric acid (111 ml) for $5\frac{1}{2}$ hours, the cooled solution was washed with ether, and the aqueous layer was decolorised with activated charcoal and evaporated to dryness *in vacuo*. The residual colourless glass was dissolved in the minimum quantity of absolute ethanol and added dropwise to a stirred, cooled (—10°C) mixture of absolute ethanol (125 ml) and thionyl chloride (15.7 g). The resulting solution was set aside at room temperature for 1 hour, refluxed for $1\frac{1}{2}$ hours, cooled, and poured into ice-water, adjusting the pH to 9 with aqueous sodium hydroxide. The mixture was extracted with ether, and the dried extract was concentrated and distilled, giving diethyl 2-aminononanedioate (55% yield) as a colourless oil, b.p. 114—115°C/0.02—03 mm.

*B. Diethyl 2-((4,4-dimethyl-3-oxo-phenylpentyl)amino)nonanedioate*

To diethyl 2-aminononanedioate (5.18 g) was added dropwise 4,4-dimethyl-5-phenylpent-1-en-3-one (3.95 g) with cooling and stirring. The mixture was allowed to stand at room temperature for 21 hours to give diethyl 2-(4,4-dimethyl-3-oxo-5-phenylpentyl)amino)nonanedioate.

*C. Diethyl 2-(3-hydroxy-4,4-dimethyl-5-phenylpentyl)amino)nonanedioate*

The foregoing crude ketone (5.1 g) was dissolved in absolute ethanol (70 ml) and the solution was stirred in an ice-bath during the gradual addition of sodium borohydride (380 mg). The solution was stirred in the ice-bath for a further 10 minutes and then left to stand at room temperature for 5 hours. Most of the alcohol was evaporated, water was added, and the solution acidified to pH 6. The insoluble oil was extracted with ether, and the ether solution was dried and evaporated to leave diethyl 2-(3-hydroxy-4,4-dimethyl-5-phenylpentyl)amino)nonanedioate as a pale yellow oil which was used without further purification.

*D. 5-(6-Ethoxycarbonyl-hexyl)-1-(3-hydroxy-4,4-dimethyl-5-phenylpentyl)hydantoin and the corresponding acid*

A solution of the above alcohol (8.45 g) in ethanol (37.6 ml) and 2N-hydrochloric acid (18.8 ml) was stirred and cooled in ice during the dropwise addition of a solution of potassium cyanate (3.05 g) in water (5.6 ml). The mixture was allowed to stand at room temperature for 18 hours, then the alcohol was evaporated, water was added and the insoluble oil was extracted with ether. The dried ether solution was evaporated to leave a viscous oil which was heated on the steam bath for 6 hours to give 5 - (6 - ethoxycarbonylhexyl) - 1 - (3 - hydroxy - 4,4 - dimethyl - 5 - phenylpentyl)hydantoin as a viscous pale yellow oil.

This ester was added to a mixture of 2N-sodium hydroxide (25 ml) and water (60 ml) and the resulting cloudy solution was left at room temperature for 2 hours. The solution was washed with ether and the clear alkaline solution was acidified with 2N-hydrochloric acid and the precipitated oil was extracted with ether. Evaporation of the dried ether solution gave a viscous oil (6.8 g) which was chromatographed on a column of silica gel to give 5 - (6 - carboxyhexyl - 1 - (3 - hydroxy - 4,4 - dimethyl - 5 - phenylpentyl)hydantoin as a colourless viscous oil which solidified, m.p. *ca.* 115°C, shrinking from *ca.* 90°C being a mixture of diastereomers. Recrystallisation several times from a mixture of ethyl acetate and light petroleum (b.p. 60—0°C) gave one of the diastereomers as small needles, m.p. 135—137°C.

Reference Preparation 2

Preparation of 5-(6-Carboxyhexyl)-1-(3-hydroxyoctyl)hydantoin

*A. Diethyl 2-(3-tetrahydropyran-2-yloxy)octylamino)nonanedioate*

Dry ethereal hydrogen bromide, prepared from ether (200 ml) and hydrogen bromide (26.8 g) at 0°C, was added dropwise to a stirred solution of acrolein (19.15 g) in ether (100 ml) cooled to −25°C. The stirred mixture was kept at this temperature for 1 hour, allowed to come to 0°C, stirred for 1 hour, at 0°C and then added dropwise to ethereal pentyl magnesium bromide (prepared from 1-bromopentane (54 g) magnesium (8.8 g) and ether (120 ml)), maintaining constant reflux. The reaction mixture was decomposed with saturated aqueous ammonium chloride and extracted with ether, and the dried extract was concentrated and distilled, giving 1-bromo-3-hydroxyoctane as a colourless oil, b.p. 68.5—72.5°C/0.08 mm. A solution of this bromo-alcohol (20.9 g) in dihydropyran (17.0 g), was treated with *p*-toluenesulphonic acid (500 mg) in a little ether, set aside at room temperature for 18 hours, and washed with aqueous sodium bicarbonate. The organic layer was percolated through silica in 1:9 ether/hexane and the solvent was removed *in vacuo*, giving 1-bromo-3-(tetrahydropyran-2-yloxy)octane as a colourless oil, $\delta$ 0.88 (3H, triplet, —$CH_3$) and 4.62 (1H, broad, —O—*CHR*—O—). A solution of this tetrahydropyranyl intermediate (15.0 g) and diethyl 2-aminononanedioate (13.0 g) in absolute ethanol (100 ml) was refluxed for 18 hours, the ethanol was removed *in vacuo*, and the residue was diluted with water containing a slight excess of sodium carbonate. The mixture was extracted with dichloromethane, the extract was dried over sodium sulphate and evaporated, and the residue was purified by column chromatography on silica in 1:4 hexane/ether, giving diethyl 2-(3-(tetrahydropyran-2-yloxy)octylamino)nonanedioate as a colourless viscous oil, $\delta$ 0.88 (3H, triplet —$CH_3$), 2.28 (2H, triplet, —$CH_2$—$CO_2Et$), 2.61 (2H, multiplet, —$CH_2$—N), 3.20 (1H, triplet, N—CHR—$CO_2Et$), 4.13 (4H, multiplet, 2 x —O—$CH_2$—$CH_3$), 4.60 (1H, broad —O—*CHR*—O—).

The above aminodiester was alternatively prepared in the following manner. Diethyl 2-aminononanedioate (10.40 g) and oct-1-en-3-one (5.04 g) were mixed slowly at 0°C. with stirring, and set aside at room temperature for 3 hours, giving diethyl 2-(3-oxooctylamino)nonanedioate as a colourless oil, $\delta$ 2.3 (4H, multiplet, —$CH_2$—$CO_2Et$ and $NCH_2CH_2CO$—), 3.16 (1H, triplet, $EtO_2C$—*CHR*—N), 4.11 (2H, quartet, —O—$CH_2$—$CH_3$), 4.17 (2H, quartet, —O—$CH_2$—$CH_3$). A stirred solution of this ketone (13.5 g) in absolute ethanol (140 ml) was treated dropwise at 0°C with sodium borohydride (665 mg) in absolute ethanol (70 ml), then kept for $3\frac{1}{2}$ hours at room temperature, and concentrated at 40°C *in vacuo*. The residue, dissolved in water, was brought to pH 5 wirh *N*-hydrochloric acid and extracted thoroughly with chloroform, the extract was washed with water, dried, and evaporated, giving diethyl

2-(3-hydroxyoctylamino)nonanedioate as a colourless oil. Without further purification, the latter was dissolved in dihydropyran (14.0 ml), treated with ether (10 ml) followed by *p*-toluenesulphonic acid (6.72 g) in portions, and set aside at room temperature for 18 hours. The reaction solution was diluted with ether, washed with aqueous sodium carbonate then water, dried, and evaporated, and the residue was purified by column chromatography on silica in 1:4 hexane/ether, giving diethyl 2-(3-(tetrahydropyran-2-yloxy)octylamino)nonanedioate identical (n.m.r., i.r. mixed t.l.c.) with that prepared previously.

*B. 5-(6-Carboxyhexyl)-1-(3-(tetrahydropyran-2-yloxy)octyl hydantoin*

To a solution of diethyl 2-(3-(tetrahydropyran-2-yloxy)octylamino)nonanedioate (7.8 g) in ethanol (32 ml) was added a solution of potassium cyanate (3.0 g) in water (6 ml). The resulting suspension was stirred and cooled during the gradual addition of 2*N*-hydrochloric acid (16.7 ml). The solution was allowed to stand at room temperature for 22 hours, most of the ethanol was evaporated, water was added, and the insoluble oil was extracted with ether. The ether solution was washed with water, dried over magnesium sulphate, and evaporated. The yellow oil so obtained (8.0 g) was dissolved in light petroleum (b.p. 60—80°C) and the solution was refluxed for 4 hours, evaporated to dryness, and the residual oil was heated on the steambath for 2 hours to give 5 - (6 - ethoxycarbonylhexyl) - 1 - (3 - (tetrahydropyran-2-yloxy)octyl)hydantoin as a yellow oil (7.3 g) which was used without further purification.

A solution of the ester (6.2 g) in 0.5*N*-sodium hydroxide solution (80 ml) was allowed to stand at room temperature for $2\frac{1}{2}$ hours after which the solution was washed with ether, the aqueous layer was acidified with 2*N*-hydrochloric acid, and the precipitated oil was extracted with ether. The washed and dried ether estract was evaporated to give 5-(6-carboxyhexyl)-1-(3-(tetrahydropyran-2-yloxy)octyl)-hydantoin as a yellow oil.

*C. 5-(6-Carboxyhexyl)-1-(3-hydroxyoctyl hydantoin*

This tetrahydropyranyloxy compound (3.55 g) was dissolved in tetrahydrofuran (28 ml) and 5*N*-hydrochloric acid (7 ml) and the solution was left at room temperature for $3\frac{1}{2}$ hours, and then refluxed for 30 minutes. Most of the solvent was evaporated, water was added and the insoluble oil was extracted with ether. The ether solution was washed with water, dried over magnesium sulphate and evaporated to give 3.15 g of viscous yellow oil. The oil was purified by chromatography on a column of silica gel, elution first with chloroform and then with a mixture of chloroform and methanol (19:1) giving 5-(6-carboxyhexyl)-1-(3-hydroxyoctyl)hydantoin as a very viscous almost colourless oil, $\delta$ 0.89 (3H, triplet, —$CH_3$), 2.34 (2H, triplet, —$CH_2$—$CO_2H$), 2.9—4.2 (4H, complex, —$CH_2$—N, *CH*—N, *CH*—OH), *ca.* 5.6 (2H, broad, exchangeable, —$CO_2H$, —OH), *ca.* 9.0 (1H, broad, exchangeable, NH).

Using the method of Reference Preparation 1 the above identified hydantoin was also prepared via the corresponding diethyl 2-(3-hydroxyoctyl)amino)nonanedioate.

*D. Separation of Diastereomers*

The hydantoin resulting from the above described preparation was a viscous oil which by use of HPLC on a column of silica with a mixture of chloroform, methanol and acetic acid (97:2.5:0.5) as solvent was separated into two diastereomers, both of which formed small colourless needles of m.p. 76—78°C and 63—65°C respectively.

The same diastereomers were prepared by cyclisation of the corresponding diastereomers of formula (III). That is, the mixture of diastereomers of diethyl 2-[(3-hydroxyoctyl)amino]nonanedioate, prepared as in Reference Preparation 1 was dissolved in ethanol and an ethereal solution of hydrogen chloride was added. The solution was evaporated to dryness to leave the mixture of diastereomeric hydrochlorides as a viscous oil, which partly solidified on standing. Ether was added and the mass stirred and cooled to give a crystalline solid, which was collected, washed with ether, and dried. The solid was crystallised from ethyl acetate to give small colourless plates, m.p. 95—96.5°C, of a pure hydrochloride. This salt was suspended in dilute sodium hydroxide solution and shaken with ether, and the separated ether solution was washed, dried and evaporated to give one of the diastereomers (A) of diethyl 2-[(3-hydroxyoctyl)amino]nonanedioate as a colourless oil.

The ether filtrate remaining after collection of the original solid hydrochloride was evaporated to leave an oily hydrochloride, which was converted to base as described above to give the almost pure second diastereomer (B) of diethyl 2-[(3-hydroxyoctyl)amino]nonanedioate as a colourless oil.

By the method described in Reference Preparation 1, the above diastereomer (A) was converted into a single diastereomer of 5-(6-carboxyhexyl)-1-(3-hydroxyoctyl)hydantoin, which crystallised from a mixture of ethyl acetate and light petroleum (b.p. 60—80°C) as small colourless needles, m.p. 63—65°C.

Similarly the above diastereomer (B) was converted into the second diastereomer of 5-(6-carboxyhexyl)-1-(3-hydroxyoctyl)hydantoin, which crystallised from ethyl acetate light petroleum (b.p. 60—80°C) as small colourless needles, m.p. 76—78°C.

*B. Interconversion of the diastereomers*

A solution of 5-(6-carboxyhexyl)-1-(3-hydroxyoctyl)hydantoin (diastereomer of m.p. 76—78°C) (100 mg) in *N*-sodium hydroxide solution (3 ml) was allowed to stand at room temperature for 19 hours. The solution was acidified and extracted with ether, and the ether extract was washed with water, dried and evaporated to leave a viscous oil. By means of high performance liquid chromatography this oil was separated into the two diastereomers of 5-(6-carboxyhexyl)-1-(3-hydroxyoctyl)hydantoin, m.p. 76—78°C identical with the starting material (*ca.* 40 mg) and m.p. 63—65°C (*ca.* 40 mg) identical with the diastereomer (A) described above.

In similar fashion, the diastereomer of m.p. 63—65°C was converted into a mixture of approximately equal quantities of itself with the diastereomer of m.p. 76—78°C, and the pure diastereomers were isolated by means of high performance liquid chromatography.

Using the method of the preceding Reference Preparations the following were prepared:—

1a) diethyl 2-((3-oxo-3-(4-tetrahydropyranyl)propyl) amino)nonanedioate;
2a) diethyl 2-((3-oxo-3-*cis*-(4-methylcyclohexyl)propyl)amino)nonanedioate;
3a) diethyl 2-((3-oxo-3-*trans*-(4-methylcyclohexyl)propyl)amino)nonanedioate;
4a) diethyl 2-((3-oxo-3-(1-adamantyl)propyl) amino)nonanedioate;
5a) diethyl 2-((3-oxo-3-(2-thienyl)propyl) amino)nonanedioate;
6a) diethyl 2-((3-oxo-3-(2-furyl)propyl) amino)nonanedioate;
7a) diethyl 2-((3-oxo-3-(cyclopent-3-enyl)propyl) amino)nonanedioate;
8a) diethyl 2-((norbornan-2-on-3-ylmethyl) amino)nonanedioate;
9a) diethyl 2-((3-oxo-3-(3,3-dimethylcyclobutyl)propyl) amino)nonanedioate;
10a) diethyl 2-((3-oxo-3-(2,2,3,3-tetramethylcyclopropyl)propyl) amino)nonanedioate;
11a) diethyl 2-((3-oxo-3-(1-methylcyclohexyl)propyl) amino)nonanedioate;
12a) diethyl 2-((3-oxo-3-*trans*-(4-methylcyclohexyl)propyl)amino)-non-4*Z*-enedioate;
13a) diethyl 2-((3-oxo-3-*cis*-(4-methylcyclohexyl)propyl)amino)-non-4*Z*-enedioate;
14a) ethyl 2-((3-oxo-3-phenylpropyl)amino)-4-(ethoxycarbonylmethylthio)hexanoate;
15a) ethyl 2-((3-oxo-3-cycloyexylpropyl)amino)-5-ethoxycarbonylmethylthiopentanoate;
16a) ethyl 2-((3-oxo-3-cyclohexylpropyl)amino)6-ethoxycarbonylmethylthiohex-4*Z*-enoate;
17a) ethyl 2-((3-oxo-3-cyclohexylpropyl)amino)-6-ethoxycarbonylmethylthiohex-4*E*-enoate;
18a) ethyl 2-((3-oxo-3-cyclohexylpropyl)amino)-3-(3-ethoxycarbonylpropylthio)propionate;
19a) ethyl 2-(3-oxooctylamino)-3-(3-ethoxycarbonylpropylthio)-propionate;
20a) ethyl 2-((3-oxo-3-cyclopentylpropyl)amino)-3-(4-ethoxycarbonylbutylthio)propionate;
21a) diethyl 2-((3-oxo-3-(3-furyl)propyl)amino)nonanedioate;
22a) diethyl 2-((3-oxo-3-(4-benzyloxyphenyl)propyl)amino)nonanedioate; and
23a) ethyl 2-((3-oxo-3-cyclohexylpropyl)amino)-5-(2-ethoxycarbonylethylthio)pentanoate;

which were converted to:

1b) diethyl 2-((3-hydroxy-3-(4-tetrahydropyranyl)propyl)amino)-nonanedioate;
2b) diethyl 2-((3-hydroxy-3-*cis*-(4-methylcyclohexyl)propyl)amino)-nonanedioate;
3b) diethyl 2-((3-hydroxy-3-*trans*-(4-methylcyclohexyl)propyl)amino)-nonanedioate;
4b) diethyl 2-((3-hydroxy-3-(1-adamantyl)propyl)amino)nonanedioate;
5b) diethyl 2-((3-hydroxy-3-(2-thienyl)propyl)amino)nonanedioate;
6b) diethyl 2-((3-hydroxy-3-(2-furyl)propyl)amino)nonanedioate;
7b) diethyl 2-((3-hydroxy-3-(cyclopent-3-enyl)propyl)amino)nonanedioate;
8b) diethyl 2-((norbornan-2-hydroxy-3-ylmethyl)amino)nonanedioate;
9b) diethyl 2-((3-hydroxy-3-(3,3-dimethylcyclobutyl)propyl)amino)-nonanedioate;
10b) diethyl 2-((3-hydroxy-3-(2,2,3,3-tetramethylcyclopropyl)propyl)amino)nonanedioate;
11b) diethyl 2-((3-hydroxy-3-(1-methylcyclohexyl)propyl)amino)-nonanedioate;
12b) diethyl 2-((3-hydroxy-3-*trans*-(4-methylcyclohexyl)propyl)amino)-non-4*Z*enedioate;
13b) diethyl 2-((3-hydroxy-3-*cis*-(4-methylcyclohexyl)propyl)amino)-non-4*Z*-enedioate;
14b) ethyl 2-((3-hydroxy-3-phenylpropyl)amino-6-ethoxycarbonylmethylthiohexanoate;
15b) ethyl 2-((3-hydroxy-3-cyclohexylpropyl)amino)-5-ethoxycarbonylmethylthiopentanoate;
16b) ethyl 2-((3-hydroxy-3-cycloyexylpropyl)amino)-6-ethoxycarbonylmethylthiohex-4*Z*-enoate;
17b) ethyl 2-((3-hydroxy-3-cyclohexylpropyl)amino)-6-ethoxycarbonylmethylthiohex-4*E*-enoate;
18b) ethyl 2-((3-hydroxy-3-cyclohexylpropyl)amino)-3-(3-ethoxycarbonylpropylthio)propionate;
19b) ethyl 2-((3-hydroxyoctylamino)-3-(3-ethoxycarbonylpropylthio)propionate;
20b) ethyl 2-((3-hydroxy-3-cyclopentylpropyl)amino)-3-(4-ethoxycarbonylbutylthio)propionate;
21b) diethyl 2-((3-hydroxy-3-(3-furyl)propyl)amino)nonanedioate;
22b) diethyl 2-((3-hydroxy-3-(4-benzyloxyphenyl)propyl)amino)-nonanedioate; and
23b) ethyl 2-((3-hydroxy-3-cyclohexylpropyl)amino-5-(2-ethoxycarbonylethylthio)pentanoate;

from which were obtained the following hydantoins which, where indicated, were separated to provide individual diastereomers having the stated characteristics:

1c) 5-(6-carboxyhexyl)-1-(3-hydroxy-3-(4-tetrahydropyranyl)propyl)-hydantoin, 106—108°C; 101—103°C;
2c) 5-(6-carboxyhexyl)-1-(3-hydroxy-3-*cis*-(4-methylcyclohexyl)propyl)-hydantoin, 88—90°C, 98—100°C;
3c) 5-(6-carboxyhexyl)-1-(3-hydroxy-3-*trans*-(4-methylcyclohexyl)propyl)hydantoin, 87—89°C, 99—101°C;
4c) 5-(6-carboxyhexyl)-1-(3-hydroxy-3-(1-adamantyl)propyl)hydantoin, 155—157°C;
5c) 5-(6-carboxyhexyl)-1-(3-hydroxy-3-(2-thienyl)propyl)hydantoin, 78—80°C;
6c) 5-(6-carboxyhexyl)-1-(3-hydroxy-3-(2-furyl)propyl)hydantoin;
7c) 5-(6-carboxyhexyl)-1-(3-hydroxy-3-(cyclopent-3-enyl)propyl)hydantoin, 86—87°C;
8c) 5-(6-carboxyhexyl)-1-(2$\alpha$-hydroxynorbonan-3-ylmethyl)hydantoin,103—107°C, 132—136°C;
9c) 5-(6-carboxyhexyl)-1-(3-hydroxy-3-(3,3-dimethylcyclobutyl)-propyl)hydantoin, 91—92°C., 108—109°C;
10c) 5-(6-carboxyhexyl)-1-(3-hydroxy-3-(2,2,3,3-tetramethylcyclopropyl)hydantoin, 93—95°C, 135—137°C;
11c) 5-(6-carboxyhexyl)-1-(3-hydroxy-3-(1-methylcyclohexyl)propyl)hydantoin, 100—102°C, 131—132°C;
12c) 5-(6-carboxyhex-2*Z*-enyl)-1-(3-hydroxy-3-*trans*-(4-methylcyclohexyl)propyl)hydantoin, 63—65°C, 87—89°C;
13c) 5-(6-carboxyhex-2*Z*-enyl)-1-(3-hydroxy-3-*cis*-4-methylcyclohexylpropyl)hydantoin, 63—65°C, 87—89°C;
14c) 5-(4-carboxymethylthiobutyl)-1-(3-hydroxy-3-phenylpropyl)hydantoin, 101—102.5°C;
15c) 5-(3-carboxymethylthiopropyl)-1-(3-hydroxy-3-cyclohexylpropyl)hydantoin, 93—96°C;
16c) 5-(4-carboxymethylthiobut-2*Z*-enyl)-1-(3-hydroxy-3-cyclohexylpropyl)hydantoin, 117—118.5°C, 116—117°C;
17c) 5-(4-carboxymethylthiobut-2E-enyl)-1-(3-cyclohexyl-3-hydroxypropyl)hydantoin;
18c) 5-(3-carboxypropylthiomethyl)-1-(3-hydroxy-3-cyclohexylpropyl)hydantoin, 131—133°C, 108—110°C;
19c) 5-(3-carboxypropylthiomethyl)-1-(3-hydroxyoctyl)hydantoin, 44—47°C, 57—60°C;
20c) 5-(4-carboxybutylthiomethyl)-1-(3-hydroxy-3-cyclopentylpropyl)hydantoin, 80—82°C,91—93°C;
21c) 5-(6-carboxyhexyl)-1-(3-hydroxy-3-(3-furyl)propyl)hydantoin;
22c) 5-(6-carboxyhexyl)-1-(3-hydroxy-3-(4-benzyloxyphenyl)propyl)hydantoin, 70—71°C, 130—130.5°C; and
23c) 5-(3-2-carboxyethylthio)propyl)-1-(3-hydroxy-3-cyclohexylpropyl)hydantoin, 100.5—102.5°C, 95—97°C.

## Example A

*Inhibition of Platelet Aggregation*

Aggregation of platelets in 1 ml of fresh human platelet rich plasma (PRP) was monitored in a Born aggregometer.

The compound to be tested was added to the PRP at the desired concentration, and the resulting mixture incubated at 37°C for 1 minute after which platelet aggregation was stimulated by the addition of adenosine diphosphate (ADP) to a concentration of 5 $\mu$M.

The anti-aggregatory effect of the compound was assessed by measuring the percentage inhibition of platelet aggregation in the presence of the compound as compared when it was completely absent.

Each of prostaglandin $E_1$ and 5-(4-carboxymethylthiobutyl)-1-(3-cyclohexyl-3-hydroxypropyl)-hydantoin were tested in this way and the latter was found to be about 20 times more potent than $PGE_1$.

In the following examples the "Compound" was 3-(4-carboxymethylthiobutyl)-1-(3-cyclohexyl-3-hydroxypropyl)hydantoin.

## Example B

| Tablet | In one tablet |
|---|---|
| Compound | 5.0 mg |
| Lactose B.P. | 82.0 mg |
| Starch B.P. | 10.0 mg |
| Povidone B.P.C. | 2.0 mg |
| Magnesium Stearate | 1.0 mg |

The Compound, lactose and starch were mixed together. The powders were granulated using a solution of the povidone in Purified Water. The granules were dried, added with the Magnesium Stearate and compressed to produce tablets, 100 mg per tablet.

Example C

| Capsule | In one capsule |
|---|---|
| Compound | 10 mg |
| Lactose | 79 mg |
| Starch | 10 mg |
| Magnesium Stearate | 1 mg |

The powders were mixed in a powder blender and filled into hard gelatin capsules, 100 mg per capsule.

Example D

| 1 $\mu$g/ml Injection | |
|---|---|
| Compound | 100 $\mu$g |
| Water for Injection | to . . . . 100 ml |

The Compound was dissolved in the Water for Injection. The solution was sterilised by filtration through a membrane filter, 0.22 $\mu$m pore size, collecting the filtrate in a sterile receiver. Under aspetic conditions, the solution was filled into sterile glass ampoules, 1 ml per ampoule. The glass was sealed by fusion.

Example E

| 10 $\mu$g/ml Injection | |
|---|---|
| Compound | 1 mg |
| Ethyl Alcohol | 10 ml |
| Propylene Glycol | 30 ml |
| Water for Injection | to . . . . . 100 ml |

The Compound was dissolved in the Ethyl Alcohol, added with the Propylene glycol and diluted to volume with Water for Injection.

The solution was sterilised by filtration through a membrane filter, 0.22 $\mu$m pore size, collecting the filtrate in a sterile vessel. Under aspetic conditions, the solution was filled into sterile glass vials, 10 ml per vial. The vials were closed with a sterile rubber plug and secured with an aluminium collar.

Example 24

Preparation of 5-(4-carboxymethylthiobutyl)-1-(3-hydroxy-3-cycloyexylpropyl)hydantoin

*A. Diethyl 2-amino-7-thianonanedioate*

Diethyl 4-bromobutylacetamidomalonate (Rec. Trav. Chim., 1971, 874) (35.2 g) was dissolved in absolute ethanol (150 ml) and a solution of ethyl mercaptoacetate (12.0 g) in ethanolic sodium ethoxide (from 2.30 g sodium and 130 ml ethanol) added dropwise with stirring. A slight exotherm soon subsided, and the resulting cloudy mixture was stirred at room temperature for 24 hours. The mixture was evaporated to low bulk *in vacuo,* then diluted with water and extracted with chloroform. The dried extract was evaporated giving diethyl acetamido-(4-ethoxycarbonylmethylthio)butyl malonate (42.2 g) as a colourless gum. This was suspended in 10% aqueous hydrochloric acid (750 ml) and refluxed for 3 hours, cooled, then evaporated to low bulk *in vacuo,* using ethanol to remove the last traces of water azeotropically. The residual gum was dissolved in a little ethanol and added to a solution

of thionyl chloride (21.0 ml) and ethanol (300 ml) (made up at −10°C) and the solution stood at −5°C for 18 hours then refluxed for 1 hour. The cooled solution was evaporated, and the residue partitioned between chloroform and water and the aqueous layer taken to pH ~10 with sodium carbonate solution. The aqueous layer was extracted thoroughly with chloroform and the coimbined extracts dried and evaporated, giving diethyl 2-amino-7-thia-nonanedioate (25.5 g) as a pale yellow oil.

*B. Ethyl 2-((3-oxo-3-cyclohexylpropyl)amino)-6-(ethoxycarbonylmethylthio)hexanoate*

To diethyl 2-amino-7-thianonanedioate (4.16 g) was added dropwise 3-cyclohexylprop-1-en-3-one (2.07 g) with cooling and stirring. The mixture was allowed to stand at room temperature for 21 hours to give ethyl 2-((3-oxo-3-cyclohexylpropyl)amino)-6-(ethoxycarbonylmethylthio)hexanoate as a pale yellow oil.

*C. Ethyl 2-((3-hydroxy-3-cyclohexylpropyl)amino)-6-(ethoxycarbonylmethylthio)hexanoate*

The foregoing crude ketone (6.2 g) was dissolved in absolute ethanol (180 ml) and the solution was stirred in an ice-bath during the gradual addition of sodium borohydride (750 mg). The solution was stirred in the ice-bath for a further 10 minutes and then left to stand at room temperature overnight. Most of the alcohol was evaporated, water was added, and the insoluble oil was extracted with ether, and the ether solution was dried and evaporated to leave ethyl 2-((3-hydroxy-3-cyclohexylpropyl)amino)-6-(ethoxycarbonylmethylthio)hexanoate as a pale yellow oil which was used without further purification.

*D. 5-(4-Carboxymethylthiobutyl)-1-(3-hydroxy-3-cyclohexylpropyl)hydantoin*

A solution of the above alcohol (6.05 g) in ethanol (30 ml) and 2*N*-hydrochloric acid (15 ml) was stirred and cooled in ice during the dropwise addition of a solution of potassium cyanate (2.60 g) in water (8 ml). The mixture was allowed to stand at room temperature for 18 hours, then the alcohol was evaporated, water was added and the insoluble oil was extracted with ether. The dried ether solution was evaporated to leave a viscous oil which was heated on the steam bath for 19 hours to give 5-(4-ethoxycarbonylmethylthiobutyl)-1-(3-hydroxy-3-cyclohexylpropyl)-hydantoin as a viscous pale yellow oil.

A solution of this ester in 0.5 N-sodium hydroxide (75 ml) was left at room temperature for 2 hours. The solution was washed with ether and the clear alkaline solution was acidified with 2*N*-hydrochloric acid and the precipitated oil was extracted with ether. Evaporation of the dried ether solution gave a viscous oil (6.8 g) which was chromatographed on a column of silica gel to give 5-(4-carboxymethylthiobutyl)-1-(3-hydroxy-3-cyclohexylpropyl)hydantoin as a viscious oil. Separation by HPLC on a column of silica with a mixture of chloroform, methanol and acetic acid (97:2.5:0.5) gave the two pure diastereomers, one being obtained as a viscous gum and the other forming colourless crystals, m.p. 104—108°C.

The starting substances for Examples 15 to 17:—

1. diethyl 2-amino-6-thia-octanedioate;
2. diethyl 2-amino-7-thia-non-4*Z*-enoate;
3. diethyl 2-amino-7-thia-non-4*E*-enoate were obtained from:—

1. diethyl 3-chloropropylacetamidomalonate (Coll. Czech. Chem. Comm., 1968, 33, 3823);
2. diethyl 4-chlorobut-2*Z*-enylacetamidomalonate (cf. Chem. Eng. Data (1970) 205); and
3. diethyl 4-chlorobut-2*E*-enylacetamidomalonate (Chem. Eng. Data. (1970) 205);

in a manner analogous to that described for the starting substance for Example 24.

The starting substance for Example 20, *S*-(4-Ethoxycarbonylbutyl)-*L*-cysteine ethyl ester, was prepared as follows:—

*L*-cysteine ethyl ester hydrochloride (18.6 g, 0.10 mole) was added to a solution of sodium (4.6 g, 0.20 g.a.) in ethanol (320 ml., absolute), stirred 15 minutes and then the solvent removed *in vacuo*. To a solution of the residual sodium salts in dry dimethylsulphoxide (200 ml) was added ethyl 5-bromovalerate (21.0 g, 0.10 mole) in a single portion with stirring. The reaction mixture was allowed to stand at room temperature overnight, warmed on a steam bath for 5 minutes and then poured onto water (300 ml) containing sodium dihydrogen phosphate (1 g). The oil was extracted with ether (3 ×), the combined extracts washed with water and dried over $MgSO_4$. The solvent was removed *in vacuo* yield 24.7 g of product as a pale yellow oil.

*S*-(3-Ethoxycarbonyl)propyl-*L*-cysteine ethyl ester, used in Examples 18 and 19 was prepared in an analogous manner.

Diethyl 2-amino-6-thianonanedioate, used in Example 23 was prepared as follows:—

Diethyl 3-chloropropylacetamidomalonate (Coll. Czech. Chem. Comm., 1968, *33*, 3823) (18.5 g) and sodium iodide (100 mg) were dissolved in absolute ethanol (90 ml) and a solution of ethyl 3-mercaptopropionate (7.96 g) in ethanolic sodium ethoxide (from 1.37 g sodium and 75 ml ethanol) added dropwise with stirring. The solution was stirred at room temperature for 45 hours, evaporated to low volume *in vacuo* then diluted with water and extracted with chloroform. The dried extract was evaporated giving crude diethyl acetamido-3-(2-ethoxycarbonylethylthio)propylmalonate (24 g) as a

pale yellow syrup. This was suspended in 10% aqueous hydrochloric acid (470 ml) and the mixture refluxed for 3 hours, cooled, then evaporated to low bulk *in vacuo,* using ethanol to remove the last traces of water zeotropically. The residual gum was dissolved in a little ethanol and added to a solution of thionyl chloride (13.5 ml) and ethanol (200 ml) (made up at −10°C) and the solution was allowed to stand at +5°C for 18 hours, then refluxed for 1 hour. The cooled solution was evaporated and the residue partitioned between chloroform and aqueous sodium carbonate solution. The chloroform layer was separated and the aqueous layer extracted twice more with chloroform and the combined organic extracts dried and evaporated, and the residual oil subjected to column chromatography on silica. Elution with 7% methanol/chloroform gave diethyl 2-amino-6-thianonanedioate (7.0 g) as a pale yellow oil.

**Claims**

1. A compound of formula (I):

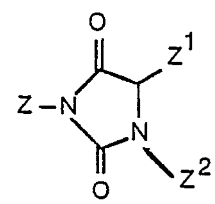

(I)

wherein Z is hydrogen or alkyl; one of $Z^1$ and $Z^2$ is represented by the group $-X-X^1-X^2-X^3$

wherein X is methylene, $X^1$ is phenylene, $-C{\equiv}C-$, *cis* or *trans* $-CH{=}CH-$ or $-CH_2-CQ_2-$ in which each Q is independently selected from hydrogen and alkyl or the two Q's together form an alkylene radical having four, five or six carbon atoms; $X^2$ is a covalent bond or a straight or branched alkylene chain having 1 to 6 carbon atoms; $X^3$ is selected from 5-tetrazolyl, carboxyl, caramoyl, hydroxymethylene and alkoxycarbonyl; and one of the methylene groups of X, $X^1$ and $X^2$ may be replaced by oxa ($-O-$) or thia ($-S-$) provided that at least one carbon atom separates the oxa or thia group from a $-C{\equiv}C-$, $-CH{=}CH-$ or $-CO-$ group or from the nitrogen of the hydantoin ring;

and the other of $Z^1$ and $Z^2$ is represented by the group $-Y-Y^1-Y^2-Y^3$

wherein Y is $-CR_2-CH_2-$ in which each R is independently selected from hydrogen and methyl; $Y^1$ is carbonyl, methylene substituted by hydroxyl or methylene substituted by hydroxyl and alkyl; $Y^2$ is a covalent bond or straight or branched alkylene chain having 1 to 7 carbon atoms optionally substituted on the carbon adjacent $Y^1$ by one or two groups each of which may be alkyl or a cyclic radical; $Y^3$ is hydrogen, hydroxy, alkoxy having 1 to 7 carbon atoms, a cyclic radical, phenyl, benzyl, phenoxy or benzyloxy, wherein each of phenyl, benzyl, phenoxy and benzyloxy may be substituted in the benzene ring by one or more groups selected from hydroxy, halo, nitro, amino, acylamino, alkenyl, alkoxy, phenyl, benzyloxy and alkyl which may itself be substituted by one or more halo groups; or $Y^2$ and $Y^3$ together form an alkyl group having 1 to 7 carbon atoms of which at least one hydrogen is replaced by fluoro; or Y is a bond, $-CH_2-$, or $-CH_2{\cdot}CH_2-$ and $Y^1$, $Y^2$ and $Y^3$ taken together form a cycloalkyl or bicycloalkyl group substituted by a hydroxyl group; provided that:

(A) either

(i) X or $X^1$ includes an oxa group; or

(ii) one of X, $X^1$ and $X^2$ includes a thia group; or

(B) when the values of X, $X^1$ and $X^2$ are other than as defined in A, then

(i) at least one of $Y^2$ and $Y^3$ includes a cyclic radical other than an unsubstituted cycloalkyl group having from 3 to 10 carbon atoms or an unsubstituted bicycloalkyl group having from 4 to 10 carbon atoms; or

(ii) $Y^2$ and $Y^3$ taken together form an alkyl group of from 1 to 7 carbon atoms of which at least one hydrogen is replaced by fluoro; or

(iii) $Y^1$, $Y^2$ and $Y^3$ taken together form a hydroxy-substituted bicycloalkyl group; or

(iv) $Y^3$ includes a benzene ring substituted by benzyloxy;

in which the term cyclic radical means the monovalent radical derived by loss of a ring hydrogen atom from a monocyclic or polycyclic compound having from 3 to 12 ring atoms selected from carbon, nitrogen, oxygen and sulphur, which compound may be saturated or unsaturated and may be further substituted by one or more alkyl groups, but excluding phenyl.

2. A compound according to claim 1, wherein $Y^1$, $Y^2$ and $Y^3$ taken together form a cycloalkyl or bicycloalkyl group substituted by a hydroxyl group which has three carbon atoms separating it from the hydantoin ring.

3. A compound of claim 1, wherein in formula (I) Z is hydrogen

one of $Z^1$ and $Z^2$ is a group $-X-X^1-X^2-X^3$ wherein

X is methylene

$X^1$ is $-C{\equiv}C-$, *cis* or *trans* $-CH{=}CH-$ or $-CH_2-CH_2-$;

$X^2$ is a covalent bond or a straight or branched alkylene chain having 1 to 6 carbon atoms optionally having one of its methylene groups replaced by oxa ($-O-$) or thia ($-S-$) provided that at least one carbon atom separates the oxa or thia from a $-C{\equiv}C-$, $-CH{=}CH-$ or $-CO-$ group; and

$X^3$ is selected from carboxyl, carbamoyl, hydroxymethylene and alkoxycarbonyl;

and the other of $Z^1$ and $z^2$ is a group $-Y-Y^1-Y^2-Y^3$ wherein

Y, $Y^1$, $Y^2$ and $Y^3$ are as defined in claim 1.

4. A compound of claim 1 wherein

Z is hydrogen

$Z^1$ is a group $-X-X^1-X^2-X^3$ wherein

X is methylene

$X^1$ is *cis* or *trans* $-CH{=}CH-$ or $-CH_2-CH_2-$

$X^2$ is $-CH_2{\cdot}CH_2-$, $-CH_2{\cdot}O{\cdot}CH_2-$ or $-CH_2{\cdot}S{\cdot}CH_2-$; and

$X^3$ is carboxyl or alkoxycarbonyl; and

$Z^2$ is a group $-Y-Y^1-Y^2-Y^3$ wherein

Y is $-CH_2-CH_2-$

$Y^1$ is methylene substituted by hydroxyl or methylene substituted by hydroxyl and alkyl;

$Y^2$ is a covalent bond or straight or branched alkylene having 1 to 7 carbon atoms optionally substituted in the carbon adjacent $Y^1$ by one or two groups each of which may be alkyl or a cyclic radical;

$Y^3$ is hydrogen, alkoxy of 1 to 7 carbon atoms or a cyclic radical.

5. A compound of claim 4 wherein in formula (I) a cyclic radical is cycloalkyl of 3 to 10 carbon atoms.

6. A compound of claim 5, wherein cycloalkyl is cyclopentyl or cyclohexyl.

7. A compound selected from the compounds as defined in claim 1:
5-(4-carboxymethylthiobutyl)-1-(3-hydroxy-3-cyclohexylpropyl)-hydantoin;
5-(4-carboxymethylthiobut-2-E-enyl)-1-(3-hydroxy-3-cyclohexylpropyl)-hydantoin;
5-(4-carboxymethylthiobutyl)-1-(3-hydroxy-3-phenylpropyl)-hydantoin;
5-(3-carboxymethylthiopropyl)-1-(3-hydroxy-3-cyclohexylpropyl)-hydantoin;
5-(4-carboxymethylthiobut-2z-enyl)-1-(3-hydroxy-3-cyclohexyl-1-propyl)-hydantoin;
5-(4-carboxymethylthiobut-2E-enyl)-1-(3-cyclohexyl-3-hydroxypropyl)-hydantoin;
5-(3-carboxypropylthiomethyl)-1-(3-hydroxy-3-cyclohexylpropyl)-hydantoin;
5-(3-carboxypropylthiomethyl)-1-(3-hydroxyoctyl)-hydantoin;
5-(4-carboxybutylthiomethyl)-1-(3-hydroxy-3-cyclopentylpropyl)-hydantoin;
5-(3-(2-carboxyethylthio)propyl)-1-(3-hydroxy-3-cyclohexylpropyl)-hdantoin;
and alkyl esters thereof.

8. A compound selected from the compounds as defined in claim 1:
5-(6-carboxyhexyl)-1-(3-hydroxy-3-(4-tetrahydropyranyl)propyl)-hydantoin;
5-(6-carboxyhexyl)-1-(3-hydroxy-3-*cis*-(4-methylcyclohexyl)-propyl)-hydantoin;
5-(6-carboxyhexyl)-1-(3-hydroxy-3-*trans*-(4-methylcyclohexyl)-propyl)-hydantoin;
5-(6-carboxyhexyl)-1-(3-hydroxy-3-(1-adamantyl)propyl)-hydantoin;
5-(6-carboxyhexyl)-1-(3-hydroxy-3-(2-thienyl)propyl)-hydantoin;
5-(6-carboxyhexyl)-1-(3-hydroxy-3-(2-furyl)propyl)-hydantoin;
5-(6-carboxyhexyl)-1-(3-hydroxy-3-(cyclopent-3-enyl)propyl)-hydantoin;
5-(6-carboxyhexyl)-1-(3-hydroxy-3-(3,3-dimethylcyclobutyl)-propyl)-hydantoin;
5-(6-carboxyhexyl)-1-(3-hydroxy-3-(2,2,3,3-tetramethyl-cyclopropyl)-hydantoin;
5-(6-carboxyhexyl)-1-(3-hydroxy-3-(1-methylcyclohexyl)-propyl)-hydantoin;
5-(6-carboxyhexyl)-1-(3-hydroxy-3-(3-furyl)propyl)-hydantoin;
and alkyl esters thereof.

9. A compound selected from the compounds as defined in claim 1:
5-(6-carboxyhexyl)-1-(2$\alpha$-hydroxynorbonan-3-ylmethyl)-hydantoin;
and alkyl esters thereof.

10. A compound selected from the compounds as defined in claim 1;
5-(6-carboxyhexyl)-1-(3-hydroxy-3-(4-benzyloxyphenyl)-propyl)-hydantoin;
and alkyl esters thereof.

11. The diastereomer of a compound claimed in any preceding claim which diastereomer is shown to be the less polar by thin layer chromatography using silica gel and a solvent system of chloroform:methanol:acetic acid in the proportions of 90:5:5.

12. A method of preparing a compound of formula (I) as defined in any of claims 1 to 11 comprising cyclisation of a compound of formula (II):

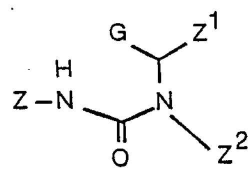

(II)

wherein G is carboxyl or a derivative thereof, and each of Z, $Z^1$ and $Z^2$ has the same meaning as in formula (I).

13. A compound of formula:

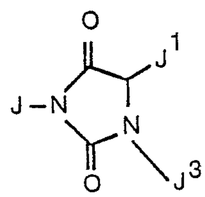

(X)

wherein J is hydrogen or alkyl, $J^1$ is hydrogen or $Z^1$ as defined in claim 1 and $J^2$ is hydrogen or $Z^2$ as defined in claim 1, provided that one and only one of $J^1$ and $J^2$ is hydrogen and provided that

(A) either
(i) X or $X^1$ includes an oxa group; or
(ii) one of X, $X^1$ and $X^2$ includes a thia group; or

(B) when the values of X, $X^1$ and $X^2$ are other than as defined in A, then
(i) at least one of $Y^2$ and $Y^3$ includes a cyclic radical other than an unsubstituted cycloalkyl group having from 3 to 10 carbon atoms or an unsubstituted bicycloalkyl group having from 4 to 10 carbon atoms; or
(ii) $Y^2$ and $Y^3$ taken together form an alkyl group of from 1 to 7 carbon atoms of which at least one hydrogen is replaced by fluoro; or
(iii) $Y^1$, $Y^2$ and $Y^3$ taken together form a hydroxy-substituted bicycloalkyl group; or
(iv) $Y^3$ includes a benzene ring substituted by benzyloxy in which the term cyclic radical means the monovalent radical derived by loss of a ring hydrogen atom from a monocyclic or polycyclic compound having from 3 to 12 ring atoms selected from carbon, nitrogen, oxygen and sulphur, which compound may be saturated or unsaturated and may be further substituted by one or more alkyl groups, but excluding phenyl.

14. A pharmaceutical composition comprising a compound of formula (I) as defined in any of claims 1 to 11 in association with a pharmaceutically acceptable carrier.

15. A composition as claimed in claim 14 in the form of a unit dose.

16. A composition as claimed in claim 15 comprising from 0.1 to 50 mg of a compound of formula (I).

17. A compound of formula (I) according to any of claims 1 to 11 for use in the inhibition of aggregation of blood platelets.

18. A compound of formula (I) according to any of claims 1 to 11 for use in the treatment or prophylaxis of thrombosis in a mammal or mammalian tissue.

19. A compound of formula (I) according to any of claims 1 to 11 for use in the lowering of blood pressure in a mammal including man.

20. A compound of formula (I) according to any of claims 1 to 11 for use in the treatment or prophylaxis of gastric lesions in a mammal.

**Patentansprüche**

1. Verbindung der Formel I

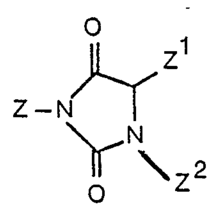

(I)

worin Z ein Wasserstoffatom oder einen Alkylrest bedeutet; einer der Reste $Z^1$ und $Z^2$ durch den Rest $—X—X^1—X^2—X^3$ angegeben wird,

worin X für Methylen steht, $X^1$ Phenylen, —C≡C—, cis- oder trans- —CH=CH— oder —$CH_2$—$CQ_2$— ist, worin jedes Q unabhängig unter Wasserstoff und Alkyl ausgewählt ist oder die beiden Q zusammen eine Alkylenrest mit vier, fünf oder sechs Kohlenstoffatomen bilden; $X^2$ eine kovalente Bindung oder eine gerade oder verzweigte Alkylenkette mit 1 bis 6 Kohlenstoffatomen bedeutet; $X^3$ unter 5-Tetrazolyl, Carboxyl, Carbamoyl, Hydroxymethylen und Alkoxycarbonyl ausgewählt ist; und eine der Methylengruppen von X, $X^1$ und $X^2$ durch Oxa (—O—) oder Thia (—S—) ersetzt sein kann, mit der Maßgabe, daß mindestens ein Kohlenstoffatom die Oxa- oder Thia-Gruppe von einer —C≡C—, —CH=CH— oder —CO— Gruppe oder vom Stickstoff des Hydantoinringes trennt;

und der andere der Reste $Z^1$ und $Z^2$ durch die Gruppe —Y—$Y^1$—$Y^2$—$Y^3$ dargestellt wird,

worin Y der Rest —$CR_2$—$CH_2$— ist, in dem jedes R unabhängig unter Wasserstoff und Methyl ausgewählt ist; $Y^1$ Carbonyl, durch Hydroxyl substituiertes Methylen oder durch Hydroxyl und Alkyl substituiertes Methylen ist; $Y^2$ eine kovalente Bindung oder eine gerade oder verzweigte Alkylenkette mit 1 bis 7 Kohlenstoffatomen ist, die wahlweise am $Y^1$ benachbarten Kohlenstoffatom durch ein oder zwei Reste substituiert ist, wovon jeder ein Alkylrest oder ein cyclischer Rest sein kann; $Y^3$ Wasserstoff, Hydroxy, Alkoxy mit 1 bis 7 Kohlenstoffatomen, ein cyclischer Rest, Phenyl, Benzyl, Phenoxy oder Benzyloxy ist, worin jeder Phenyl-, Benzyl-, Phenoxy- und Benzyloxyrest in dem Benzolring durch einen oder mehrere Reste, ausgewählt unter Hydroxy, Halogen, Nitro, Amino, Acylamino, Alkenyl, Alkoxy, Phenyl, Benzyloxy und Alkyl, das selbst durch ein oder mehrere Halogenatome substituiert sein kann, substituiert sein kann; oder $Y^2$ und $Y^3$ zusammen einen Alkylrest mit 1 bis 7 Kohlenstoffatomen bilden, wobei mindestens ein Wasserstoffatom durch Fluor ersetzt worden ist; oder Y eine Bindung, —$CH_2$— oder —$CH_2 \cdot CH_2$— ist und $Y^1$, $Y^2$ und $Y^3$ zusammen einen Cycloalkyl- oder Bicycloalkylrest bilden, der durch eine Hydroxylgruppe substituiert ist, mit der Maßgabe, daß:

(A) entweder

(i) X oder $X^1$ einen Oxarest umfaßt oder

(ii) einer der Reste X, $X^1$ und $X^2$ eine Thiagruppe umfaßt;

oder

(B) wenn die Angaben von X, $X^1$ und $X^2$ andere als gemäß der Definition in (A) sind,

(i) mindestens einer der Reste $Y^2$ und $Y^3$ einen anderen cyclischen Rest umfaßt, als einen unsubstituierten Cycloalkylrest mit 3 bis 10 Kohlenstoffatomen oder einen unsubstituierten Bicycloalkylrest mit 4 bis 10 Kohlenstoffatomen; oder

(ii) $Y^2$ und $Y^3$ zusammen einen Alkylrest mit 1 bis 7 Kohlenstoffatomen bilden, wovon mindestens ein Wasserstoffatom durch Fluor ersetzt ist; oder

(iii) $Y^1$, $Y^2$ und $Y^3$ zusammen einen hydroxysubstituierten Bicycloalkylrest bilden; oder

(iv) $Y^3$ einen Benzolring umfaßt, der durch Benzyloxy substituiert ist:

worin der Begriff cyclischer Rest den monovalenten Rest bedeutet, der durch Verlust eines Ringwasserstoffatoms von einer monocyclischen oder polycyclischen Verbindung mit 3 bis 12 Ringatomen, ausgewählt unter Kohlenstoff, Stickstoff, Sauerstoff und Schwefel, erhalten wird, wobei die Verbindung gesättigt oder ungesättigt sein kann und ferner durch einen oder mehrere Alkylgruppen substituiert sein kann, jedoch unter Ausschluß von Phenyl.

2. Verbindung nach Anspruch 1, worin $Y^1$, $Y^2$ und $Y^3$ zusammen einen Cycloalkyl- oder Bicycloalkylrest bilden, der durch einen Hydroxylrest substituiert ist, der drei Kohlenstoffatome aufweist, die ihn von dem Hydantoinring trennen.

3. Verbindung nach Anspruch 1, worin in Formel (I) Z ein Wasserstoffatom ist, einer der Reste $Z^1$ und $Z^2$ eine Gruppe —X—$X^1$—$X^2$—$X^3$ ist, worin X für Methylen steht, $X^1$ für —C≡C—, cis- oder trans- —CH=CH— oder —$CH_2$—$CH_2$— steht, $X^2$ eine kovalente Bindung oder eine gerade oder verzweigte Alkylenkette mit 1 bis 6 Kohlenstoffatomen darstellt, worin wahlweise eine ihrer Methylengruppen durch Oxa (—O—) oder Thia (—S—) ersetzt ist; mit der Maßgabe, daß mindestens ein Kohlenstoffatom die Oxagruppe oder Thiagruppe von einer —C≡C—, —CH=CH— oder —CO-Gruppe trennt, und $X^3$ unter Carboxyl, Carbamoyl, Hydroxymethylen und Alkoxycarbonyl ausgewählt ist, und der andere Rest von $Z^1$ und $Z^2$ ein Rest —Y—$Y^1$—$Y^2$—$Y^3$ ist, worin Y, $Y^1$, $Y^2$ und $Y^3$ der in Anspruch 1 angegebenen Bedeutung entsprechen.

4. Verbindung nach Anspruch 1, worin Z ein Wasserstoffatom bedeutet, $Z^1$ ein Rest —X—$X^1$—$X^2$—$X^3$ ist, worin X für Methylen steht, $X^1$ cis- oder trans- —CH=CH— oder —$CH_2$—$CH_2$- bedeutet, $X^2$ für —$CH_2 \cdot CH_2$—, —$CH_2 \cdot O \cdot CH_2$— oder —$CH_2 \cdot S \cdot CH_2$— steht, und $X^3$ Carboxyl oder Alkoxycarbonyl darstellt, und $Z^2$ ein Rest —Y—$Y^1$—$Y^2$—$Y^3$ ist, worin Y für —$CH_2$—$CH_2$— steht, $Y^1$ durch Hydroxyl substituiertes Methylen oder durch Hydroxyl und Alkyl substituiertes Methylen bedeutet, $Y^2$ eine kovalente Bindung oder ein geradkettiges oder verzweigtes Alkylen mit 1 bis 7 Kohlenstoffatomen darstellt, welches wahlweise an dem an $Y^1$ angrenzenden Kohlenstoffatom durch ein oder zwei Reste substituiert ist, wovon jeder ein Alkylrest oder ein cyclischer Rest sein kann, $Y^3$ Wasserstoff, Alkoxy mit 1 bis 7 Kohlenstoffatomen oder ein cyclischer Rest ist.

5. Verbindung nach Anspruch 4, worin in Formel (I) ein cyclischer Rest ein Cycloalkyl mit 3 bis 10 Kohlenstoffatomen ist.

6. Verbindung nach Anspruch 5, worin der Cycloalkylrest Cyclopentyl oder Cyclohexyl ist.

7. Verbindung, ausgewählt unter den Verbindungen gemäß der Definition in Anspruch 1:

5-(4-Carboxymethylthiobutyl)-1-(3-hydroxy-3-cyclohexylpropyl)-hydantoin;
5-(4-Carboxymethylthiobut-2-E-enyl)-1-(3-hydroxy-3-cyclohexylpropyl)-hydantoin;
5-(4-Carboxymethylthiobutyl)-1-(3-hydroxy-3-phenylpropyl)-hydantoin;
5-(3-Carboxymethylthiopropyl)-1-(3-hydroxy-3-cyclohexylpropyl)-hydantoin;
5-(4-Carboxymethylthiobut-2z-enyl)-1-(3-hydroxy-3-cyclohexyl-1-propyl)-hydantoin;
5-(4-Carboxymethylthiobut-2E-enyl)-1-(3-cyclohexyl-3-hydroxypropyl)-hydantoin;
5-(3-Carboxypropylthiomethyl)-1-(3-hydroxy-3-cyclohexylpropyl)-hydantoin;
5-(3-Carboxypropylthiomethyl)-1-(3-hydroxyoctyl)-hydantoin;
5-(4-Carboxybutylthiomethyl)-1-(3-hydroxy-3-cyclopentylpropyl)-hydantoin;
5-(3-(2-Carboxyethylthio)propyl)-1-(3-hydroxy-3-cyclohexylpropyl)-hydantoin
und deren Alkylester.

8. Verbindung, ausgewählt unter den Verbindungen gemäß der Definition in Anspruch 1:
5-(6-Carboxyhexyl)-1-(3-hydroxy-3-(4-tetrahydropyranyl)-propyl)-hydantoin;
5-(6-Carboxyhexyl)-1-(3-hydroxy-3-cis-(4-methylcyclohexyl)-propyl)-hydantoin;
5-(6-Carboxyhexyl)-1-(3-hydroxy-3-trans-(4-methylcyclohexyl)-propyl)-hydantoin;
5-(6-Carboxyhexyl)-1-(3-hydroxy-3-(1-adamantyl)propyl)-hydantoin;
5-(6-Carboxyhexyl)-1-(3-hydroxy-3-(2-thienyl)propyl)-hydantoin;
5-(6-Carboxyhexyl)-1-(3-hydroxy-3-(2-furyl)propyl)-hydantoin;
5-(6-Carboxyhexyl)-1-(3-hydroxy-3-(cyclopent-3-enyl)-propyl)-hydantoin;
5-(6-Carboxyhexyl)-1-(3-hydroxy-3-(3,3-dimethylcyclobutyl)propyl)-hydantoin;
5-(6-Carboxyhexyl)-1-(3-hydroxy-3-(2,2,3,3-tetramethyl)cyclopropyl)-hydantoin;
5-(6-Carboxyhexyl)-1-(3-hydroxy-3-(1-methylcyclohexyl)propyl)-hydantoin;
5-(6-Carboxyhexyl)-1-(3-hydroxy-3-(3-furyl)propyl)-hydantoin
und deren Alkylester.

9. Verbindung, ausgewählt unter den Verbindungen gemäß der Definition in Anspruch 1:
5-(6-Carboxyhexyl)-1-(2α-hydroxynorbonan-3-ylmethyl)-hydantoin
und deren Alkylester.

10. Verbindung, ausgewählt unter den Verbindungen gemäß der Definition in Anspruch 1:
5-(6-Carboxyhexyl)-1-(3-hydroxy-3-(4-benzyloxyphenyl)propyl)-hydantoin
und deren Alkylester.

11. Diastereomer einer Verbindung nach einem der voranstehenden Ansprüche, welches das weniger polare Diastereomere ist, wie durch Dünnschicht-Chromatographie nachgewiesen wird, wobei Silicagel und ein Lösungsmittel-system von Chloroform:Methanol:Essigsäure in Verhältnissen von 90:5:5 angewendet wird.

12. Verfahren zur Herstellung einer Verbindung der Formel (I) gemäß der Definition nach einem der Ansprüche 1 bis 11, wobei eine Verbindung der Formel (II)

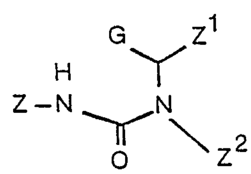

(II)

worin G Carboxyl oder ein Derivat davon ist und jeder der Reste Z, $Z^1$ und $Z^2$ die gleiche Bedeutung wie in Formel (I) besitzt, cyclisiert wird.

13. Verbindung der Formel

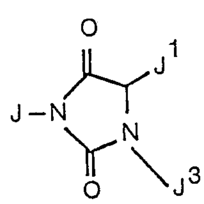

worin J ein Wasserstoffatom oder einen Alkylrest darstellt, $J^1$ ein Wasserstoffatom oder $Z^1$ gemäß der Definition in Anspruch 1 ist, und $J^2$ ein Wasserstoffatom ist oder $Z^2$ gemäß der Definition in Anspruch 1 ist, mit der Maßgabe, daß ein und lediglich ein Rest $J^1$ und $J^2$ Wasserstoff ist und mit der Maßgabe, daß

(A) entweder
(i) X oder $X^1$ eine Oxagruppe umfaßt, oder
(ii) einer der Reste X, $X^1$ und $X^2$ eine Thiagruppe umfaßt,

oder

(B) wenn die Angaben von X, $X^1$ und $X^2$ andere als gemäß der Definition in (a) sind,

(i) mindestens einer der Reste $Y^2$ und $Y^3$ einen anderen cyclischen Rest umfaßt, als einen unsubstituierten Cycloalkylrest mit 3 bis 10 Kohlenstoffatomen oder einen unsubstituierten Bicycloalkylrest mit 4 bis 10 Kohlenstoffatomen, oder

(ii) $Y^2$ und $Y^3$ zusammen einen Alkylrest mit 1 bis 7 Kohlenstoffatomen bilden, wobei mindestens ein Wasserstoffatom durch Fluor ersetzt ist, oder

(iii) $Y^1$, $Y^2$ und $Y^3$ zusammen einen Hydroxy-substituierten Bicycloalkylrest bilden, oder

(iv) $Y^3$ einen Benzolring umfaßt, der durch Benzyloxy substituiert ist,

worin der Begriff cyclischer Rest den monovalenten Rest bedeutet, der durch Verlust eines Ringwasserstoffatoms von einer monocyclischen oder polycyclischen Verbindung mit 3 bis 12 Ringatomen, ausgewählt unter Kohlenstoff, Stickstoff, Sauerstoff und Schwefel, erhalten wird, wobei die Verbindung gesättigt oder ungesättigt sein kann und ferner durch einer oder mehrere Alkylgruppen substituiert sein kann, jedoch unter Ausschluß von Phenyl.

14. Pharmazeutische Zusammensetzung, enthaltend eine Verbindung nach Formel (I) gemäß der Definition in einem der Ansprüche 1 bis 11 in Zusammenstellung mit einem pharmazeutisch verträglichen Träger.

15. Zusammensetzung gemäß Anspruch 14 in Form einer Einheitsdosis.

16. Zusammensetzung nach Anspruch 15, enthaltend 0, 1 bis 50 mg einer Verbindung nach Formel (I).

17. Verbindung nach Formel (I) gemäß einem der Ansprüche 1 bis 11 zur Verwendung bei der Inhibierung der Aggregation von Blutplättchen.

18. Verbindung nach Formel (I) gemäß einem der Ansprüche 1 bis 11 zur Verwendung in der Behandlung oder Prophylaxe von Thrombose bei einem Säugetier oder Säugetier-Gewebe.

19. Verbindung nach Formel (I) gemäß einem der Ansprüche 1 bis 11 zur Verwendung bei der Verringerung des Blutdruckes bei einem Säugetier einschließlich des Menschen.

20. Verbindung nach Formel (I) gemäß einem der Ansprüche 1 bis 11 zur Verwendung in der Behandlung oder Prophylaxe von Magenschädigungen bei einem Säugetier.

**Revendications**

1. Composé de formule (I):

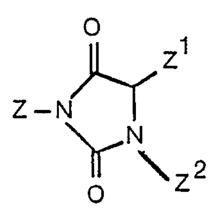

(I)

où Z représente un atome d'hydrogène ou radical alcoyle; l'un d'entre $Z^1$ et $Z^2$ représente le radical —X—$X^1$—$X^2$—$X^3$

dans lequel X représente un radical méthylène, $X^1$ représente un radical phénylène, un radical —C≡C—, un radical *cis* ou *trans* —CH=CH—, ou un radical —$CH_2$—$CQ_2$— dont chaque Q représente indépendamment un atome d'hydrogène ou radical alcoyle ou les deux Q ensemble représentent un radical alcoylène comptant 4, 5 ou 6 atomes de carbone; $X^2$ représente une liaison covalente ou un radical alcoylène en chaîne droite ou ramifiée comptant 1 à 6 atomes de carbone; $X^3$ est choisi entre un radical 5-tétrazolyle, un radical carboxyle, un radical carbamoyle, un radical hydroxyméthylène et un radical alcoxycarbonyle; et l'un des radicaux méthylène de X, $X^1$ et $X^2$ peut être remplacé par un radical oxa (—O—) ou thia (—S—) à la condition qu'au moins un atome de carbone sépare le radical oxa ou thia d'un radical —C≡C—, —CH=CH— ou —CO— ou bien de l'atome d'azote du cycle d'hydantoïne;

et l'autre d'entre $Z^1$ et $Z^2$ représente le radical —Y—$Y^1$—$Y^2$—$Y^3$

dans lequel Y représente un radical —$CR_2$—$CH_2$— dont chaque R représente indépendamment un atome d'hydrogène ou radical méthyle; $Y^1$ représente un radical carbonyle, un radical méthylène substitué par hydroxyle ou un radical méthylène substitué par hydroxyle et alcoyle; $Y^2$ représente une liaison covalente ou un radical alcoylène en chaîne droite ou ramifiée de 1 à 7 atomes de carbone éventuellement substitué sur l'atome de carbone adjacent à $Y^1$ par un ou deux radicaux dont chacun peut être un radical alcoyle ou un radical cyclique; $Y^3$ représente un atome d'hydrogène, un radical hydroxyle, un radical alcoxy comptant 1 à 7 atomes de carbone, un radical cyclique, un radical phényle, un radical benzyle, un radical phénoxy ou un radical benzyloxy, dont chaque radical phényle, benzyle, phénoxy ou benzyloxy peut être substitué sur le cycle benzénique par un ou plusieurs radicaux choisis entre hydroxyle, halo, nitro, amino, acylamino, alcényle, alcoxy, phényle, benzyloxy et alcoyle pouvant lui-même être substitué par un ou plusieurs radicaux halo, ou bien $Y^2$ et $Y^3$ représentent ensemble un radical alcoyle de 1 à 7 atomes de carbone dont au moins un atome d'hydrogène est remplacé par un radical fluoro; ou bien Y représente une liaison, un radical —$CH_2$—, ou un radical —$CH_2 \cdot CH_2$— et $Y^1$,

$Y^2$ et $Y^3$ pris ensemble forment un radical cycloalcoyle ou bicycloalcoyle substitué par un radical hydroxyle; avec la restriction que:

(A) soit
(i) X ou $X^1$ comprend un radical oxa, ou
(ii) l'un d'entre X, $X^1$ et $X^2$ comprend un radical thia,
soit

(B) lorsque les significations de X, $X^1$ et $X^2$ sont autres que celles définies en A, alors
(i) au moins l'un d'entre $Y^2$ et $Y^3$ comprend un radical cyclique autre qu'un radical cycloalcoyle non substitué comptant 3 à 10 atomes de carbone ou qu'un radical bicycloalcoyle non substitué comptant 4 à 10 atomes de carbone, ou bien
(ii) $Y^2$ et $Y^3$ pris ensemble forment un radical alcoyle comptant 1 à 7 atomes de carbone dont au moins un atome d'hydrogène est remplacé par un radical fluoro, ou bien
(iii) $Y^1$, $Y^2$ et $Y^3$ pris ensemble forment un radical bicycloalcoyle hydroxy-substitué, ou bien
(iv) $Y^3$ comprend un cycle benzénique substitué par benzoyloxy:
étant entendu que le terme radical cyclique désigne le radical monovalent issu par perte d'un atome d'hydrogène du cycle d'un composé monocyclique ou polycyclique comptant 3 à 12 atomes de cycle choisis entre les atomes de carbone, d'azote, d'oxygène et de soufre, lequel composé peut être saturé ou insaturé et peut être en outre substitué par un ou plusieurs radicaux alcoyle, mais à l'exclusion du radical phényle.

2. Composé suivant la revendication 1, dans lequel $Y^1$, $Y^2$ et $Y^3$ pris ensemble représentent un radical cycloalcoyle ou bicycloalcoyle substitué par un radical hydroxyle qui comprend trois atomes de carbone le séparant du cycle d'hydantoïne.

3. Composé suivant la revendication 1, dans lequel dans la formule (I)
Z représente un atome d'hydrogène,
l'une d'entre $Z^1$ et $Z^2$ représente un radical $-X-X^1-X^2-X^3$
dans lequel X représente un radical méthylène, $X^1$ représente un radical $-C\equiv C-$, un radical *cis* ou *trans* $-CH=CH-$ ou un radical $-CH_2-CH_2-$; $X^2$ représente une liaison covalente ou un radical alcoylène en chaîne droite ou ramifiée comptant 1 à 6 atomes de carbone dont l'un des radicaux méthylène est éventuellement remplacé par un radical oxa ($-O-$) ou thia ($-S-$) à la condition qu'au moins un atome de carbone sépare le radical oxa ou thia d'un radical $-C\equiv C-$, $-CH=CH-$ ou $-CO-$, et $X^3$ représente un radical carboxyle, un radical carbamoyle, un radical hydroxyméthylène ou un radical alcoxycarbonyle,
et l'autre d'entre $Z^1$ et $Z^2$ représente un radical $-Y-Y^1-Y^2-Y^3$
dans lequel Y, $Y^1$, $Y^2$ et $Y^3$ sont tels que définis dans la revendication 1.

4. Composé suivant la revendication 1, dans lequel Z représente un atome d'hydrogène;
$Z^1$ représente un radical $-X-X^1-X^2-X^3$
dans lequel X représente un radical méthylène, $X^1$ représente un radical *cis* ou *trans* $-CH=CH-$ ou un radical $-CH_2-CH_2-$; $X^2$ représente un radical $-CH_2{\cdot}CH_2-$, un radical $-CH_2{\cdot}O{\cdot}CH_2-$ ou un radical $-CH_2.S.CH_2-$, et $X^3$ représente un radical carboxyle ou alcoxycarbonyle et
$Z^2$ représente un radical $-Y-Y^1-Y^2-Y^3$ dans lequel Y représente un radical $-CH_2-CH_2-$, $Y^1$ représente un radical méthylène substitué par hydroxyle ou un radical méthylène substitué par hydroxyle et alcoyle; $Y^2$ représente une liaison covalente ou un radical alcoylène en chaîne droite ou ramifiée comptant 1 à 7 atomes de carbone éventuellement substitué sur l'atome de carbone adjacent à $Y^1$ par un ou deux radicaux dont chacun peut être un radical alcoyle ou un radical cyclique; $Y^3$ représente un atome d'hydrogène, un radical alcoxy comptant 1 à 7 atomes de carbone ou un radical cyclique;

5. Composé suivant la revendication 4, dans lequel dans la formule (I) un radical cyclique est un radical cycloalcoyle comptant 3 à 10 atomes de carbone.

6. Composé suivant la revendication 5, dans lequel le radical cycloalcoyle est un radical cyclopentyle ou cyclohexyle.

7. Composé choisi parmi les composés tels que définis dans la revendication 1:
5-(4-carboxyméthylthiobutyl)-1-(3-hydroxy-3-cyclohexylpropyl)hydantoïne;
5-(4-carboxyméthylthiobut-2E-ényl)-1-(3-hydroxy-3-cyclohexylpropyl)hydantoïne;
5-(4-carboxyméthylthiobutyl)-1-(3-hydroxy-3-phénylpropyl)hydantoïne;
5-(3-carboxyméthylthiopropyl)-1-(3-hydroxy-3-cyclohexylpropyl)hydantoïne;
5-(4-carboxyméthylthiobut-2Z-ényl)-1-(3-hydroxy-3-cyclohexyl-1-propyl)hydantoïne;
5-(4-carboxyméthylthiobut-2E-ényl)-1-(3-cyclohexyl-3-hydroxypropyl)hydantoïne;
5-(3-carboxypropylthiométhyl)-1-(3-hydroxy-3-cyclohexylpropyl)hydantoïne;
5-(3-carboxypropylthiométhyl)-1-(3-hydroxyoctyl)-hydantoïne;
5-(4-carboxybutylthiométhyl)-1-(3-hydroxy-3-cyclopentylpropyl)hydantoïne;
5-(3-(2-carboxyéthylthio)propyl)-1-(3-hydroxy-3-cyclohexylpropyl)hydantoïne;
et ses esters alcoyliques.

8. Composé choisi parmi les composés tels que définis dans la revendication 1:

5-(6-carboxyhexyl)-1-(3-hydroxy-3-(4-tétrahydropyranyl)propyl)hydantoïne;
5-(6-carboxyhexyl)-1-(3-hydroxy-3-*cis*-(4-méthylcyclohexyl)-propyl)hydantoïne;
5-(6-carboxyhexyl)-1-(3-hydroxy-3-*trans*-(4-méthylcyclohexyl)-propyl)hydantoïne;
5-(6-carboxyhexyl)-1-(3-hydroxy-3-(1-adamantyl)propyl)hydantoïne;
5-(6-carboxyhexyl)-1-(3-hydroxy-3-(2-thiényl)propyl)hydantoïne;
5-(6-carboxyhexyl)-1-(3-hydroxy-3-(2-furyl)propyl)hydantoïne;
5-(6-carboxyhexyl)-1-(3-hydroxy-3-(cyclopent-3-ényl)propyl)hydantoïne;
5-(6-carboxyhexyl)-1-(3-hydroxy-3-(3,3-diméthylcyclobutyl)propyl)hydantoïne;
5-(6-carboxyhexyl)-1-(3-hydroxy-3-(2,2,3,3-tetramétylcyclopropyl)propyl)hydantoïne;
5-(6-carboxyhexyl)-1-(3-hydroxy-3-(1-méthylcyclohexyl)propyl)hydantoïne;
5-(6-carboxyhexyl)-1-(3-hydroxy-3-(3-furyl)propyl)hydantoïne;
et ses esters alcoyliques.

9. Composé choisi parmi les composés tels que définis dans la revendication 1:
5-(6-carboxyhexyl)-1-(2$\alpha$-hydroxynorbornan-3-ylméthyl)hydantoïne;
et ses esters alcoyliques.

10. Composé choisi parmi les composés tels que définis dans la revendication 1:
5-(6-carboxyhexyl)-1-(3-hydroxy-3-(4-benzyloxyphényl)propyl)hydantoïne;
et ses esters alcoyliques.

11. Diastéréoisomère d'un composé suivant l'une quelconque des revendications précédentes, lequel diastéréoisomère s'est révélé être le moins polaire à la chromatographie en couche mince sur du gel de silica avec un système solvant chloroforme:méthanol:acide acétique dans les proportions 90:5:5.

12. Procédé de préparation d'un composé de formule (I) tel que défini dans l'une quelconque des revendications 1 à 11, qui comprend la cyclisation d'un composé de formule (II):

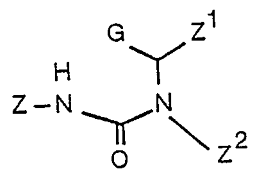

(II)

où G représente un radical carboxyle ou un dérivé de celui-ci et chacun d'entre Z, $Z^1$ et $Z^2$ a la même signification que dans la formule (I).

13. Composé de formule:

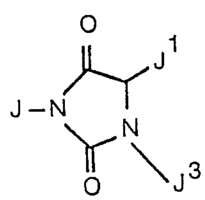

où J représente un atome d'hydrogène ou un radical alcoyle, $J^1$ représente un atome d'hydrogène ou radical $Z^1$ tel que défini dans la revendication 1 et $J^2$ représente un atome d'hydrogène ou radical $Z^2$ tel que défini dans la revendication 1, avec la restriction que l'un d'entre $J^1$ et $J^2$ et un seul représente un atome d'hydrogène et avec la restriction que

(A) soit
(i) X ou $X^1$ comprend un radical oxa, ou
(ii) l'un d'entre X, $X^1$ et $X^2$ comprend un radical thia,
soit

(B) lorsque les significations de X, $X^1$ et $X^2$ sont autres que celles définies en A, alors
(i) au moins l'un d'entre $Y^2$ et $Y^3$ comprend un radical cyclique autre qu'un radical cycloalcoyle non substitué comptant 3 à 10 atomes de carbone ou qu'un radical bicycloalcoyle non substitué comptant 4 à 10 atomes de carbone, ou bien
(ii) $Y^2$ et $Y^3$ pris ensemble forment un radical alcoyle comptant 1 à 7 atomes de carbone dont au moins un atome d'hydrogène est remplacé par un radical fluoro, ou bien
(iii) $Y^1$, $Y^2$ et $Y^3$ pris ensemble forment un radical bicycloalcoyle hydroxy-substitué, ou bien
(iv) $Y^3$ comprend un cycle benzénique substitué par benzyloxy:
étant entendu que le terme radical cyclique désigne le radical monovalent issu par perte d'un atome d'hydrogène du cycle d'un composé monocyclique ou polycyclique comptant 3 à 12 atomes de cycle choisis entre les atomes de carbone, d'azote, d'oxygène et de soufre, lequel composé peut être saturé

ou insaturé et peut être en outre substitué par un ou plusieurs radicaux alcoyle, mais à l'exclusion du radical phényle.

14. Composition pharmaceutique, comprenant un composé de formule (I) tel que défini dans l'une quelconque des revendications 1 à 11 en association avec un excipient pharmaceutiquement acceptable.

15. Composition suivant la revendication 14, sous forme de dose unitaire.

16. Composition suivant la revendication 15, comprenant 0,1 à 50 mg d'un composé de formule (I).

17. Composé de formule (I) suivant l'une quelconque des revendications 1 à 11, à utiliser pour inhiber l'agrégation des plaquettes du sang.

18. Composé de formule (I) suivant l'une quelconque des revendications 1 à 11, à utiliser pour le traitement ou la phrophylaxie de la thrombose chez un mammifère ou dans un tissu de mammifère.

19. Composé de formule (I) suivant l'une quelconque des revendications 1 à 11, à utiliser pour abaisser la tension artérielle chez un mammifère y compris l'homme.

20. Composé de formule (I) suivant l'une quelconque des revendications 1 à 11, à utiliser pour le traitement ou la phrophylaxie des lésions gastriques chez un mammifère.